# EUROPEAN PATENT SPECIFICATION

(11) **EP 4 486 401 B1**
(45) Date of publication and mention of the grant of the patent: **07.10.2026**
(21) Application number: 23707988.4
(22) Date of filing: 03.03.2023
(51) Int. Cl.: A61L 2/03

(54) **APPARATUS AND METHOD FOR REMOVAL OF BIOFILM FROM METALLIC DEVICES**
GERÄT UND VERFAHREN ZUR ENTFERNUNG VON BIOFILM AUS METALLISCHEN VORRICHTUNGEN
APPAREIL ET PROCÉDÉ D'ÉLIMINATION DE BIOFILM DE DISPOSITIFS MÉTALLIQUES

(30) Priority: 04.03.2022 EP 22382204
(43) Date of publication of application: 08.01.2025
(73) Proprietor: AMESPMTECH Center, 08980 Sant Feliu de Llobregat (ES); Innovative Minds S.L, 08783 Masquefa (Barcelona) (ES)
(72) Inventor: FONT VIZCARRA, Lluís, 08783 Masquefa (Barcelona) (ES); BERMÚDEZ CASTEL, Adrián, 08783 Masquefa (Barcelona) (ES); CALERO MARTÍNEZ, Jose Antonio, 08980 Sant Feliu de Llobregat (ES)
(74) Representative: Hoffmann Eitle
(86) International application number: PCT/EP2023/055513
(87) International publication number: WO 2023/166216

(56) References cited:
- EP-A1- 3 763 326
- CN-B- 105 209 080
- TW-B- I 643 604

## Description

### FIELD

The present disclosure relates to an apparatus and method for removal of biofilm and/or bacteria from a metallic medical device.

### BACKGROUND

Infection, whether acute or chronic, is a common complication following the implantation of a prosthesis or osteosynthesis material in a patient. This complication has a high morbidity rate, as well as a substantial mortality rate and the available treatments have a high economic cost.

Despite the many advances made in recent years in sterility measures and in systemic and local prophylactic antibiotic therapy, the infection rate continues to be between 0.3 and 25% depending on the type of intervention and the size of the implant used. Although the exact pathophysiology of orthopaedic hardware infection is not clear, it is known that any infection of orthopaedic hardware is preceded by: 1) contamination of the surgical site, 2) bacterial adhesion and 3) biofilm formation on the prosthetic material.

It is the presence of this biofilm that makes it necessary, after several weeks of infection, to remove the metal material in order to eliminate the entire bacterial load and thus cure the infection, as the methods currently available cannot destroy all the biofilm present on the surface of the implant.

The antibacterial usefulness of electric fields has been known since the beginning of the last century: a method to sterilise milk using low frequency alternating current (AC) is known. In science fields other than medicine, such as industrial water and food processing, high voltage electric fields are routinely used to "sterilise" or kill various micro-organisms and pathogens.

It has further been demonstrated the ability of electric fields to enhance the bactericidal effect of certain antibiotics. Since then, multiple in-vitro studies have emerged, demonstrating the capacity of low-energy electric fields (alternating and direct current) to inhibit bacterial growth of different strains of S. aureus, P. aeruginosa and S. epidermidis. It has also been demonstrated in rabbits the effectiveness of electric fields for the treatment of osteomyelitis caused by S. epidermidis (US5312813).

However, the application of a DC generates, among other things, the electrolysis of water, which in turn generates changes in the pH of the medium, free radicals and the release of various ions that can be cytotoxic, particularly for prolonged exposure of a DC current. In addition, there is an electro-corrosion effect (on the anode) which makes its direct application on an orthopaedic implant impossible. This is why the applicability of this current on the human body could only be carried out under very controlled conditions, so that the applicability of its studies is very limited.

Various studies have been published that demonstrate their capacity to modify electrostatic forces, altering bacterial adherence to biomaterials or even deconstructing the biofilm already formed and adhered (US 6,663,634 B2).

US 2013/0041238A1), discloses a catheter (urinary or intravascular) resistant to infection thanks to the use of electric fields.

Electric fields can be applied to the surface of the medical device in order to repel biofilms from the surface by ionic forces. However, this method is not able to repel all of the bacteria on the surface of the medical device and the process of removing the biofilm may take several hours or longer. Therefore, the electric fields affect the liquid solution (synovial fluid or blood) where the metallic implant is placed and cause the coagulation of the medium and the formation of bacterial lumps.

Another proposed method involves placing the medical device in an electrolytic solution and applying a direct electric current to the solution, wherein the electrolysis reaction generates pH changes and the formation of antibacterial molecules such as HCl which have antimicrobial properties. This process directly reduces the viability of bacteria in the medium, and kills the bacteria but again, the medical device must be placed in the electrolytic medium for several hours or days to kill the bacteria in biofilms and the potential cytotoxic effect on host cells is not controlled.

CN105209080B discloses a treatment system to clean a biofilm contaminated component, particularly to clean bone implant surfaces or dental implants contaminated by bacteria with a conductive element that can be brought into electrical contact with the component you need treatment and that is connected to a first pole of an electrical supply unit, characterized by a cannula for means provided to extract a treatment liquid, whose interior conductively joins electricity with the second pole of the power supply unit.

There is therefore a need to provide a device for removing biofilm from, or reducing bacteria adhesion to, medical devices more effectively. There is also a need to provide a device for removing biofilm from, or reducing bacteria adhesion to medical devices in which the duration of the removal process is shortened.

### SUMMARY OF THE INVENTION

According to a first aspect of the disclosure, there is provided an *in vitro* method of removing biofilm from, or reducing bacterial adhesion to, a metallic medical device external to the human or animal body, comprising: connecting a first electrode to a surface of the medical device; exposing a target area on the surface of the medical device to an electrolytic fluid; placing a second electrode proximal to the target area; providing electrical power between the first and second electrode such that electrolysis occurs in the electrolytic fluid; wherein the electrical power comprises a plurality of electrical pulses, wherein the potential difference between the first and second electrode is is at least 1V; and wherein the current density applied in each electrical pulse is up to 15A per cm² of the target area.

In a preferred embodiment, the current density applied is between 0.1 and 15 A per cm2 of the target area, preferably between 0.1 and 5 A per cm2 of the target area. In another preferred embodiment, the current density applied is between 0.2 and 2 A per cm2 of the target area.

According to the first aspect of the invention, there is provided another in vitro method of removing biofilm from, or reducing bacterial adhesion to, a metallic medical device external to the human or animal body, comprising: connecting a first electrode to a surface of the medical device; exposing a target area on the surface of the medical device to an electrolytic fluid; placing a second electrode proximal to the target area; providing electrical power between the first and second electrode such that electrolysis occurs in the electrolytic fluid; wherein the electrical power comprises a plurality of electrical pulses, wherein the potential difference between the first and second electrode is at least 1V; wherein the current applied in each electrical pulse is up to 2A, and wherein each electrical pulse has a duration of less than 1 second.

In a preferred embodiment, the step of providing electrical power between the first and second electrodes comprises providing a potential difference between 1V and 50V, preferably between 10V and 30V, and the step of providing electrical power between the first and second electrodes further comprises providing a current between 0.5A and 1A.

In another preferred embodiment, each electrical pulse has a duration between 0.05 and 0.2 seconds.

In another preferred embodiment of any of the in vitro methods of the first aspect of the invention, the second electrode is an anode comprising a sacrificial metal, preferably comprised of silver, and/or wherein the electrolytic fluid comprises one or more of a disinfecting agent, antibacterial agent or antibiotic agent; or the second electrode is an anode comprising a non-corrodible metal and free from sacrificial metals, wherein the method further comprises isolating bacteria from the electrolytic fluid after the electrical power is provided.

According to a second aspect of the disclosure, there is provided an apparatus for removal of biofilm from a metallic medical device, comprising: a fluid output connectable to a source of electrolytic fluid, the fluid output for supplying the electrolytic fluid to wash a target area of the medical device; a first electrode for electrically connecting to the medical device; the second electrode configured to be placed proximal to the fluid output; and an electric power source for supplying electrical power between the first and second electrodes; wherein the electrical power source is configured to provide intermittent electrical pulses between the first and second electrode such that electrolysis occurs at the surface of the metallic device, wherein the potential difference between the first and second electrode is at least 1V and wherein the current density applied in each electrical pulse is up to 15 A per cm² of the target area.

In a preferred embodiment, the electrical power source is configured to provide intermittent electrical pulses with current density applied is between 0.1 and 15 A per cm2 of the target area, preferably between 0.1 and 5 per cm2 of the target area.

In another preferred embodiment, the electrical power source is configured to provide intermittent electrical pulses with current density applied is between 0.2 and 2 A per cm2 of the target area.

According to the second aspect of the invention, there is provided another apparatus for removal of biofilm from a metallic medical device, comprising: a fluid output connectable to a source of electrolytic fluid, the fluid output for supplying the electrolytic fluid to wash a target area of the medical device; a first electrode for electrically connecting to the medical device; the second electrode configured to be placed proximal to the fluid output; and an electric power source for supplying electrical power between the first and second electrodes; wherein the electrical power source is configured to provide intermittent electrical pulses between the first and second electrode such that electrolysis occurs at the surface of the metallic device, wherein the potential difference between the first and second electrode is at least 1V; and wherein the current applied in each electrical pulse is up to 2A, and wherein each electrical pulse has a duration of less than 1 second.

In a preferred embodiment, the electrical power source is configured to provide intermittent electrical pulses with a potential difference between 1V and 50V, and preferably between 10V and 30V, and a current between the first and second electrodes between 0.5A and 1A.

In another preferred embodiment, the electrical power source is configured to provide each electrical pulse with a duration between 0.05 and 0.2 seconds.

In another preferred embodiment of any of the apparatus of the second aspect of the invention, the apparatus further comprises a controller configured to control the supply of electrolytic fluid from the source of fluid to the fluid output.

In another preferred embodiment, the fluid output and/or the first electrode and/or the second electrode are removably connectable to the apparatus via a detachment mechanism.

In another preferred embodiment, the fluid output comprises a tubular body, optionally wherein the tubular body is curved so that the longitudinal axis of the tubular body at a proximal end is offset from the longitudinal axis of the tubular body at a distal end by between greater than 0 and 90 degrees, or wherein the fluid output comprises a porous sponge or a conical cap at its distal end.

In another preferred embodiment, the first or second electrode and the fluid output are provided on a single detachable body connectable to the apparatus, more preferably wherein both the first and second electrode are provided on the single detachable body.

In another preferred embodiment, the power source is configured to supply an intermittent direct current between the first and second electrodes.

In another preferred embodiment, the apparatus further comprises a source of electrolytic fluid connected to the fluid applicator, wherein the fluid is a saline solution; optionally wherein the fluid comprises one or more of a disinfecting agent, antibacterial agent or antibiotic agent.

In another preferred embodiment, the apparatus further comprises a pump configured to provide the electrolytic fluid to the fluid output at a predetermined pressure.

In another preferred embodiment, the fluid output and the first and/or second electrode are configured to be connected to a handheld portion, and the handheld portion comprises a user actuated element for actuating the supply of fluid and/or electrical power.

### BRIEF DESCRIPTION OF THE DRAWINGS

Embodiments will now be explained in detail, by way of non-limiting example only, with reference to the accompanying figures described below.
Fig. 1A shows a schematic diagram of an apparatus for removal of biofilm from a metallic medical device according to one or more embodiments;
Fig. 1B shows a schematic diagram of an apparatus for removal of biofilm from a metallic medical device according to one or more embodiments;
Fig. 2 shows a schematic diagram of an electronic controller of the apparatus according to one or more embodiments.
Figs. 3A to 3F show schematic diagrams of fluid outputs for an apparatus according to one or more embodiments;
Fig. 4 shows a schematic diagram of an apparatus for removal of biofilm from a metallic medical device according to one or more embodiments;
Fig. 5A shows a controller configured to provide electric power to the apparatus according to one or more embodiments;
Fig. 5B shows another controller configured to provide electric power to the apparatus according to one or more embodiments;
Fig. 5C shows a fluid output configured to be used in conjunction with the controller shown in Fig. 5B;
Fig. 6 shows a graph of fluid pressure and current amplitude generated by an apparatus according to one or more embodiments; and
Figs. 7A to 7C show experimental data of the reduction in bacteria on a metallic device using a variety of methods according to one or more embodiments.
Figs. 8A to 8D show experimental images of an *in vitro* experiment of the reduction in bacteria on a metallic device using a method according to one or more embodiments.
Figs. 9A to 9D show experimental images of an *in vivo* experiment of the reduction in bacteria on a medical device implant using a method according to one or more embodiments.
Figs. 10A to 10G show experimental images of another *in vitro* experiment of the reduction in bacteria on a metallic device using a state-of-the-art method and a method according to one or more embodiments.

### DETAILED DESCRIPTION

The present invention relates to a method and apparatus for removal of biofilm or reducing adhesion of bacteria on a metallic medical device, which is configured to apply electrical pulses between two electrodes when one electrode is electrically connected to the metallic medical device, and when the metallic device is exposed to an electrolytic fluid. The application of the electrical pulses (specifically DC pulses) to the metallic device reduces bacterial attachment to a target area of the medical device by forming bubbles (in the case where the medical device is electrically connected to the cathode and the electrolytic solution is a saline solution, the bubbles are hydrogen bubbles) as a product of electrolysis directly on the surface of the metallic device, which mechanically lifts the bacteria or biofilm from the surface of the metallic device. The apparatus may be configured to rinse or wash the device with an intermittent supply of the electrolytic fluid to a target area of the metallic device. The intermittent supply of fluid to the target area further washes the bacteria which has been detached from the surface by the electrical pulses, and inhibits electrocoagulation and the formation of bacterial clumps.

As disclosed herein, the term "electrolytic fluid" may be considered as any liquid which contains electrolytes such that electrolysis occurs when a DC voltage is applied between a cathode and anode exposed to the electrolytic fluid. Therefore, the electrolytic fluid may be a saline solution or any other type of buffer solution.

As disclosed herein, the term "expose to electrolytic fluid" may be considered as any manner of providing the electrolytic fluid to the exposed element. This may include submerging the element in the electrolytic fluid or providing the electrolytic fluid to the exposed element by a flow of electrolytic fluid, for example by continuous or intermittent flow of the electrolytic fluid onto the element.

As disclosed herein, the term "metallic medical device" may include any medical device which includes at least a portion made of metal. The metallic device may be entirely or partly made of metal. The term "medical device" may be defined as any device which interfaces with the human or animal body in use. Medical devices may include medical implants, prosthesis or any other device configured to be placed on or in the human or animal body.

As disclosed herein, the term "sacrificial metal" may be considered as any metal whose surface dissolves into an electrolytic fluid when acting as an anode during electrolysis.

As disclosed herein, the term "non-corrodible metal" may be considered as any metal whose surface does not dissolve into an electrolytic fluid when acting as an anode during electrolysis.

The methods disclosed herein may be used to removing biofilm from, or reducing bacteria adhesion to a medical device in vivo or in vitro. In other words, the methods disclosed herein may be used to removing biofilm from, or reducing bacteria adhesion to a medical device whilst it is in or on a human or animal body, or a medical device which is external to the human or animal body.

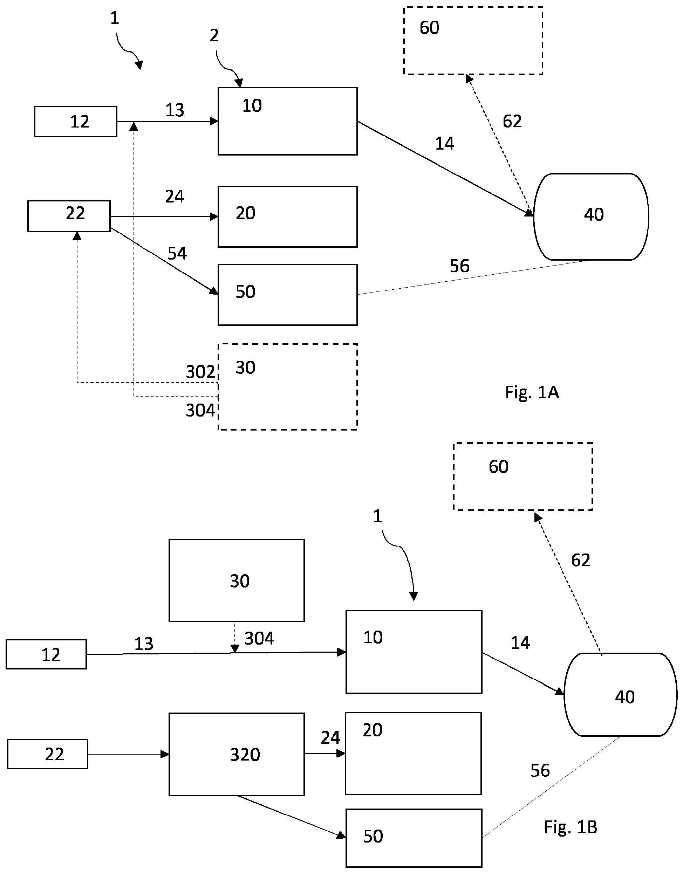

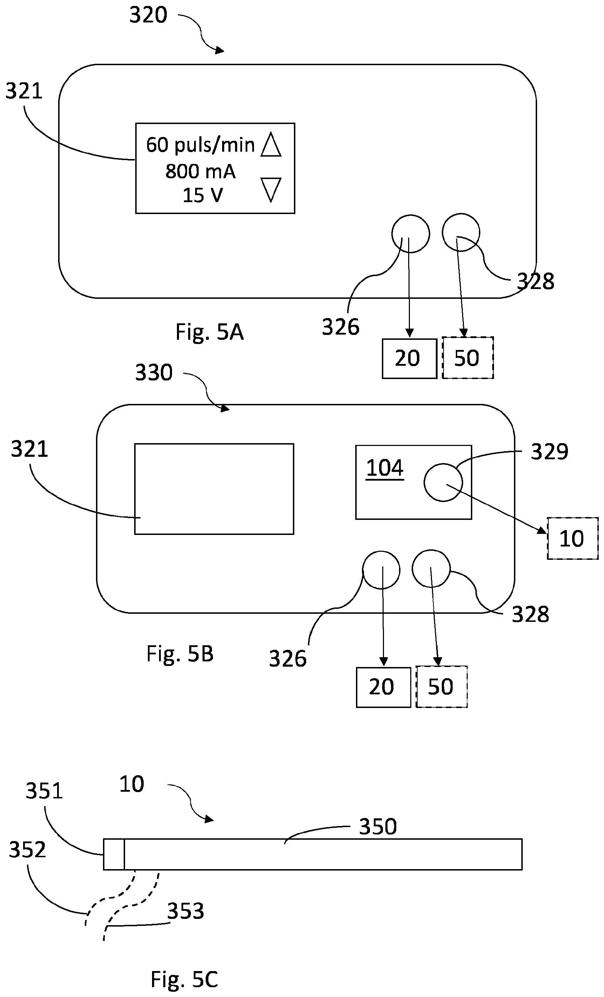

Fig. 1A shows a schematic diagram of an apparatus 1 for removing a biofilm or reducing bacteria adhesion to a metallic medical device 40. The apparatus comprises a fluid output 10, a first electrode 50 for electrically connecting to the metallic device 40, a second electrode 20, an electrical power source 22 and optionally a controller 30. The fluid output 10 is connectable to a fluid source 12 of electrolytic fluid and is configured to supply the electrolytic fluid 14 from fluid source 12 via flow path 13 to wash a target area of the medical device 40 with the electrolytic fluid. The electrode 20 is locatable proximal to the fluid output 10 and connectable to an electrical power source 22. The electrical power source 22 is connectable to the first and second electrodes 50, 20 via connections 54, 24. The apparatus 1 is configured to supply electrical power 24 between the electrodes 20, 50 such that electrolysis may occur between the electrodes 20, 50 in the presence of an electrolytic fluid. The controller 30 may be configured to control the supply of the fluid 14 from the source of fluid 12 to the fluid output 10 and may also control the supply of electrical power 24 to the electrode 20. Alternatively, a separate controller may be provided to control the supply of electrical power 24 to the electrode 20, for example as shown in Fig. 5A, or there may be no external controller provided for either the fluid source 12 or the power source 22. Furthermore, the controller 30 may be configured to supply the fluid 14 intermittently or continuously. In use, the electrolysis causes bubbles to be released from the surface at the target area of the medical device 40 which mechanically detaches bacteria from the surface of the medical device 40. In embodiments where the fluid is provided intermittently, the intermittent provision of fluid 14 to the medical device 40 further provides intermittent washing to the target area, in order to wash the detached bacteria from the surface of the medical device 40. Intermittent washing of the surface of the medical device 40 prevents the removed bacteria 40 from coagulating or forming bacterial clumps, for example on the electrode 20, on a part of the target area, or a medium in which the medical device 40 is placed. It is noted that in the devices and methods disclosed herein, the step of washing the medical device 40 may occur simultaneously to the step of applying the electric power to cause electrolysis, or the steps may occur sequentially. For example, in some embodiments the medical device 40 may be submerged in an electrolytic fluid whilst the electric power is supplied to cause the electrolysis. Subsequently, the medical device 40 may be washed with fluid from the fluid source 12. In other embodiments, the washing and electrical power may be supplied simultaneously so that electrolytic fluid both washes the device and provides the electrolytic fluid for electrolysis simultaneously.

The fluid output 10 may be connectable to any suitable fluid source 12. For example, the fluid output 10 may be connected via one or more conduits forming a flow path from a fluid reservoir to the fluid output 10. Additional components for regulating the flow of fluid from the fluid source 12 to the fluid output 10 may be provided. For example, one or more pumps or valves may be provided in order to selectively provide fluid 14 to the fluid output 10 at a desired pressure or fluid flow rate. In other embodiments, the fluid source 12 may provide the required pressure without the use of a pump. In a preferred embodiment, fluid may be provided to the fluid output 10 via a peristaltic pump so that the pump parts are not exposed to the fluid. Accordingly, the pump may be re-used between washes. The connection may be a permanent one, i.e. the conduits are integral with the fluid source 12 and the fluid output 10, or it may be detachable. For example, the conduits may be attached to the fluid source 12 and/or the fluid output 10 by a detach mechanism, such as a screw thread connection, frictional fit or stab connection, as known in the art.

The first and second electrodes 50, 20 may be connectable to any suitable power source 22. For example, the electrodes 50, 20 may be connected via electrical wiring forming an electrical connection between the power source 22 and the electrodes 50, 20. Additional electronic components may be situated between the power source 22 and the electrodes 50, 20 to regulate the power supplied by the power source 22 to the electrodes 50, 20. For example, the electrodes 50, 20 may be connected to the power source 22 by a power converter, such as any DC-DC, AC-DC, DC-AC or AC-AC power converter. The power source 22 may comprise a mains power source, one or more batteries, a constant voltage or constant current power source or the like. The electrical connection between the electrodes 50, 20 and the power source 22 be a permanent one, i.e. the electrical wiring forming the connection may be integral with the electrodes 50, 20 and/or the power source 22, or it may be detachable. For example, electrical wiring forming the connection may be attached to the power source 22 and/or the electrode 50, 20 by a detach mechanism, such as a screw thread connection, frictional fit, plug or stab connection, as known in the art.

The electrical power source 22 is configured to provide intermittent electrical pulses between the first and second electrode such that electrolysis occurs at the surface of the metallic device 40, wherein the current applied in each electrical pulse is sufficient to cause bubbles to escape the surface of the metallic device during the electrical pulse. When the potential between the electrodes is greater than the decomposition potential required for electrolysis to occur, and the current applied in each electrical pulse is sufficient to cause bubbles to escape the surface of the metallic device during the electrical pulse, bacteria or biofilms on the surface of the metallic device are lifted away from the surface of the device by the bubbles. It will be appreciated that the current and potential difference required of each electrical pulse so that bubbles escape the surface of the metallic device will depend on the apparatus used, including the surface area of the electrodes, the materials for the electrodes used, the type of electrolytic fluid and so on. For a given apparatus according to the invention, it can be verified by visual confirmation whether the current supplied for each electrical pulse is sufficient to release bubbles. For example, the medical device may be submerged in a container containing the electrolytic fluid, and it can be visually verified if bubbles escape the surface of the medical device for each pulse.

It is noted that the decomposition potential, defined as the minimum voltage between the anode and cathode (first and second electrode) needed for electrolysis to occur, depends on the oxidation and reduction reactions occurring at the electrodes and therefore depends on the electrolyte solution and the electrode metals chosen. For a saline solution, the decomposition potential is between 1V and 2V. Above the decomposition potential, the gas production rate at the electrodes increases proportionally with the current flow, according to Faraday's law. For a saline solution, a current of between greater than 0A and 2A is sufficient for bubbles to escape the surface of the medical device for each pulse. Accordingly, when a saline solution is used, the following voltage and current ranges are preferred:
- A voltage of at least 1V, preferably between 1V and 50V and more preferably between 10V to 30V;
- A current passing through the medical device of up to 2A, preferably between 0.5 and 1A.

It is however noted that these preferred values are also feasible for other electrolytic fluids such as buffer solutions. We also note that by saying a current up to 2A it is understood that it is bigger than 0A and up to 2A, i.e. that in any case there shall be some current.

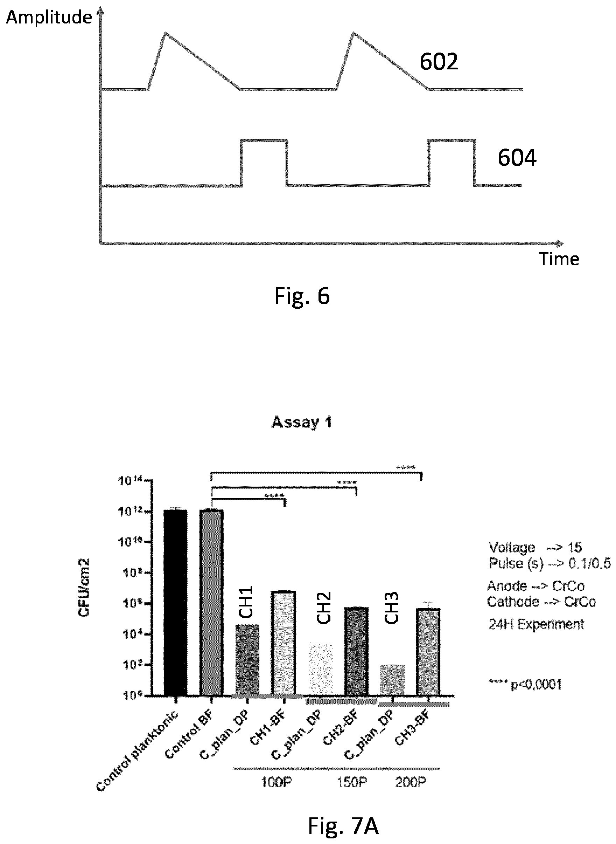

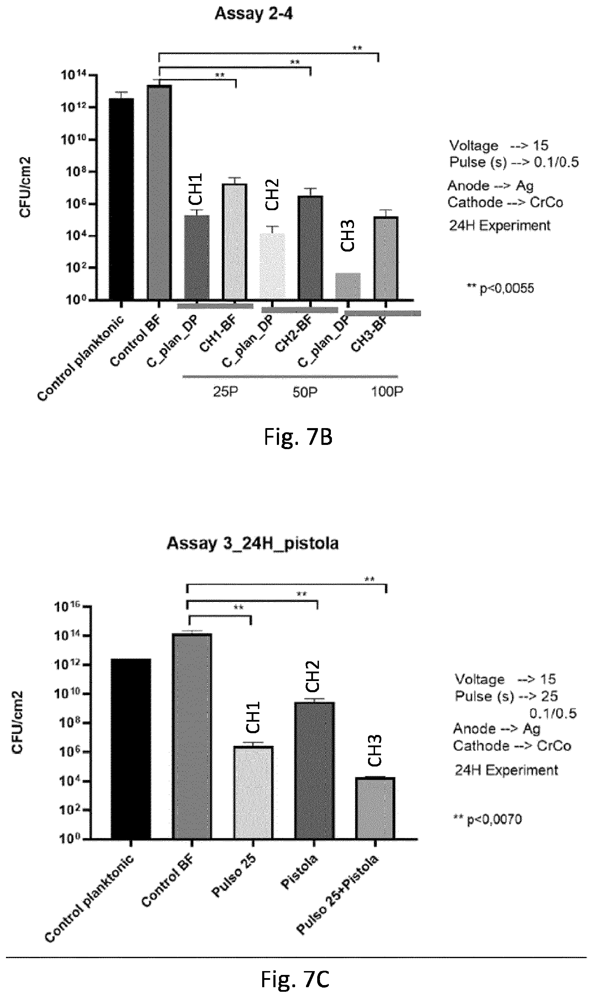

As observed in Examples 1 to 3 described herein, it has been observed that a plurality of pulses of less than 1 second in duration is sufficient to at least partially detach the biofilm. A series of 100 electrical pulses, each 0.1 seconds in duration, reduces the amount of biofilm on the substrate by several orders of magnitude (Fig. 7A, "CH1-BF"). A similar effect was observed for 25 electrical pulses, each 0.1 seconds in duration (Fig. 7B, "CH1-BF"). It has therefore been observed that applying a series of short electrical pulses (i.e. less than 1 second pulse duration) is sufficient to remove biofilm, meaning that the risks associated with a prolonged electrical treatment (for example 30 seconds or more of constant electrical current applied to the medical device), such as increased risk of prolonged exposure to free radicals or cytotoxic ions, is reduced. Applying the treatment as a series of pulses rather than a single prolonged pulse reduces the build-up of free radicals or cytotoxic ions at the medical device. The gap between electrical pulses (i.e. the time during which no current flows) may be any suitable time gap, and preferably less than 1 second, even more preferably 0.5 seconds.

The term "proximal" in relation to the second electrode 20 and the fluid output 10 should be understood as meaning the second electrode 20 and fluid output 10 are positioned sufficiently close so that the required bubbles (caused by electrolysis) escape the surface at the target area on the medical device 40 in use. The distance between the second electrode 20 and the fluid output 10 may depend on the parameters of the apparatus 1. For example, apparatus 1 may be configured to output fluid 14 from the fluid output 10 in a jet of fluid, such that the fluid output 10 is located further from the target area than the electrode 20 whilst still applying both the fluid 14 to the target area of the medical device 40 and the electric power causing electrolysis at the target area. Also, the electrical power source may be a constant current source with a maximum voltage, with larger maximum voltages allowing for the second electrode 20 to be held further away from the surface of the medical device 40 whilst still achieving the required level of current for bubbles to be released from the surface of the medical device 40 during each electrical pulse.

As disclosed herein, a fluid output may be defined as any fluid port which delivers a fluid 14 from a fluid source 12 to a target. The fluid output may for example comprise a nozzle.

The controller 30 may be any suitable controller, analogue or electronic, for controlling the supply of fluid to the fluid output 10 and/or the supply of electric power to the electrode 20. For example, the controller may be configured to control an actuatable valve which is periodically opened and closed to open and close the fluid flow path 13 between the fluid source 12 and the fluid output 10. The controller may, for example, be configured to control a pump to intermittently supply fluid to the fluid output 10 at a desired pressure. The controller 30 may also be configured to control the supply of electrical power to the electrode. For example, the controller 30 may be an electronic controller configured to open and close the electrical connections 54, 24 between the power source 22 and the electrodes 50, 20. The controller 30 may be configured to control one or more electrical components between the power source 22 and the electrodes, such as converters, variable resistors and the like. It will be appreciated that in any of the embodiments disclosed herein the controller 30 may be powered by any power source, and may be powered by the power source 22 or different power sources.

The apparatus 1 may further comprise a negative pressure source 60 configured to remove fluid from the target area. The negative pressure source 60 may be any suitable negative pressure source such as a vacuum pump, a surgical suction device or similar. The negative pressure may be transmitted from the source 60 to the target area via flexible tubing positionable at the target area of the medical device 40 and forming a fluid path 62. The negative pressure source 60 may be powered by any suitable internal or external power source, and may be powered by power source 22. The negative pressure source 60 may be configured to operate intermittently or continuously so that excess electrolytic fluid is removed from the surface of the medical device 40 intermittently or continuously. The negative pressure source 60 may be controlled by controller 30 via any suitable control connection (not shown). The controller 30 may be configured to synchronize the intermittent supply of fluid 14 with the intermittent operation of the negative pressure source 60 so that the intermittent negative pressure source 60 operates a predetermined period of time after fluid output 10 stops supplying fluid 14 to the medical device 40. The negative pressure source 60 may output the received fluid to a storage reservoir (not shown)

Fig. 1B shows a schematic diagram of an apparatus 1 for removing biofilm from, or reducing bacterial adhesion to, a medical device 40. Fig. 1B comprises the features disclosed in Fig. 1A, except a separate controller 320 is provided which is connectable to power source 22 and provides the electric power 24 between the electrodes 50, 20.

It is noted that two mechanical actions are performed by the apparatus 1 described in relation to Figs. 1A and 1B. Firstly, the bacteria is lifted of the surface of the medical device 40 by the bubbles released from the electrolysis. Secondly, the pressure of the electrolytic fluid supplied by the fluid output also causes erosion of the biofilm or bacterial layer on the surface of the medical device 40.

The power source 22 is preferably a current source. In preferred embodiments, the power source 22 is configured to provide electrical pulses of a sufficient current such that bubbles are released from the surface of the medical device 40 for each pulse. According to Faraday's first law, the mass of elements deposited at an electrode is directly proportional to the charge. Accordingly, the rate of gas production on the surface of the medical device 40 is proportional to the current provided by the power source 22. It therefore follows that the amount of current required to be supplied for each pulse depends on the area of medical device 40 over which electrolysis occurs during the pulse, which is generally determined by the cross-sectional area of the second electrode 20 facing the surface of the medical device 40. Therefore, both the cross-sectional area and the duration of the pulse will determine whether bubbles are released from the surface of the medical device 40 during each pulse. It has been observed that a current of 500mA is sufficient to observe bubbles escaping the surface during each electrical pulse for a cross-sectional area of 177mm² and a pulse duration of 0.1 seconds in duration (i.e. a current density of about 2.82mA/mm² is sufficient to cause bubbles to lift off the surface of the medical device 40). However, lower current densities could be applied for longer durations for each pulse, the pulses having sufficient duration for bubbles to escape the surface of the medical device 40. The current source preferably has a voltage range of 0 to 15V, preferably 0 to 30V and even more preferably 0 to 50V. A higher maximum voltage means that the electrode 20 can be moved further from the surface of the medical device 40 whilst the current is maintained at the required level.

As shown in Example 5, the current density may be, according to a current of 1A and for a medical device target area of 0.07cm², of 14,29A/cm², and still be safe for a subject. Therefore, although the current density may vary depending on the size of the electrode, a safe range of current density bigger than 0 A/cm² and up to 15 A/cm² may be stablished. More preferably, the current density may be comprised between 0.1 A/cm² and 5 A/cm² even more preferably between 0.2 A/cm² and 2 A/cm². The spatial dimensions are always referred to the area of the medical device over which electrolysis occurs during the pulse also known as the target area of the medical device, although in some embodiments it may be defined as the cross-sectional area of the second electrode (40) when the voltage is not enough to provide the current to all the area of the device.

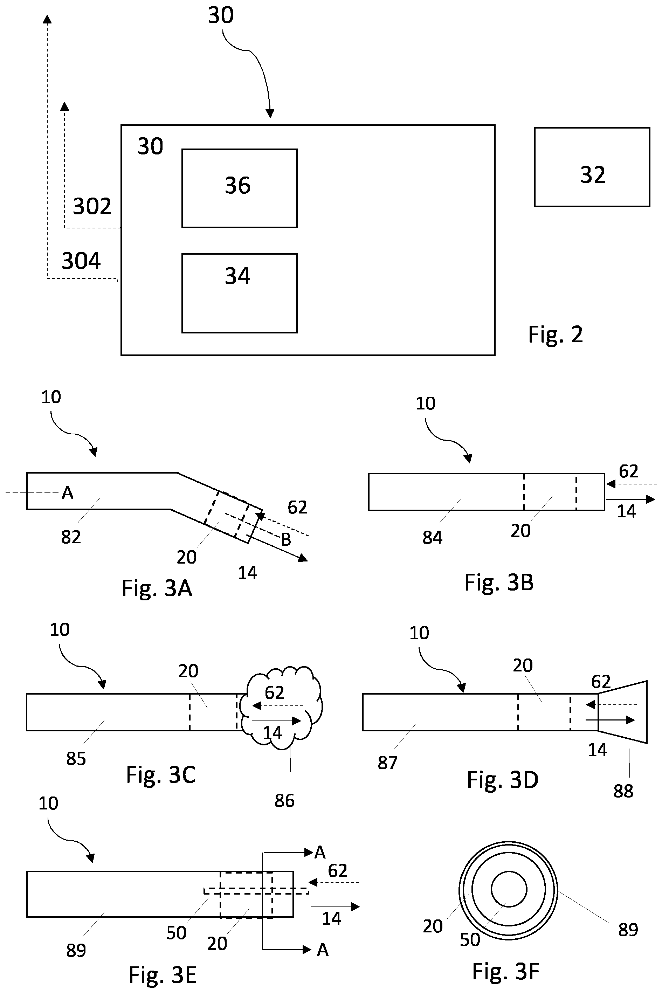

Fig. 2 shows a schematic diagram of an electronic controller 30. The electronic controller 30 comprises one or more of a memory 34 and processor 36. The memory 34 may contain instructions, which when executed using the processor 36, cause the fluid output 10 and/or the electrode 20 to be operated according to any suitable sequence including the sequences of operation disclosed herein. The controller 30 is configured to control supply of the fluid 14 to the fluid output 10 via control connection 302. The controller 30 may be configured to control supply of electrical power 24 from power source 22 via control connection 304. It is noted that control lines 302 and 304 may be physical control lines (analogue or electronic) configured to provide control signals to the fluidic components in the flow path 13 between (such as valves, pumps, pump motors and the like) and power regulating components in the electrical connection between the power source 22 and electrodes 50, 20 (such as power converters, variable resistors and the like). In other embodiments, the controller 30 may be configured to communicate with the various components wirelessly, for example via Bluetooth or any other suitable communication protocol, and the control connections 302 and 304 may be wireless. The controller 320 of Fig. 1B may have a similar configuration to control supply of electrical power to the electrodes 50, 20.

The memory 34 may comprise one or more volatile or non-volatile memory devices, such as DRAM, SRAM, flash memory, read-only memory, ferroelectric RAM, hard disk drives, floppy disks, magnetic tape, optical discs, or similar. Likewise, the processor 36 may comprise one or more processing units, such as a microprocessor, GPU, CPU, multi-core processor or similar. The controller 30 may further be implemented in software, hardware, or any combination in order to execute the sequences of operation disclosed herein.

The controller 30 may be further connected to a user interface 32 configured to select one or more parameters of the fluid supply control such as pressure, flow rate, pulse frequency and pulse duration of the fluid supply. The user interface 32 may alternatively or additionally be configured to select one or more parameters of the electric supply such as voltage, current and pulse rate and duration. The user interface 32 may comprise buttons, knobs, sliders, levers, wheels, a touchscreen, and so on, that the user can use to select the parameters. The controller 320 may be connected to the same user interface or a separate user interface to select one or more parameters of the power supply such as voltage, current, pulse rate and duration of the cleaning operation.

Fig. 3A shows a fluid output 10 according to one or more embodiments for use with apparatus 1. The fluid output 10 comprises a tubular body 82 comprising one or more fluid conduits. The one or more fluid conduits provide a fluid paths for the fluid 14 from the fluid source 12 to the target area on the medical device 40. Optionally, the one or more fluid conduits are connected to the negative pressure source 60 so that the tubular body 82 also provides a fluid path 62 for the fluid extracted by the negative pressure source 60. Optionally, the second electrode 20 is provided at or proximal to the distal tip of the tubular body 82. The tubular body 82 may comprise a single orifice at its distal end for the fluid output so that the electrolytic fluid is output as a single jet of fluid, or the tubular body 82 may comprise a plurality of orifices at its distal end so that the electrolytic fluid is output as a shower or spray of fluid. The tubular body 82 is curved so that the longitudinal axis A of the tubular body 82 at a proximal end of the tubular body is different to the longitudinal axis B of the tubular body 82 at a distal end of the tubular body 82. The angle between the longitudinal axes A and B may be a value between greater than 0 degrees and 90 degrees, and more preferably between 60 and 90 degrees. The curved form of the tubular body 82 allows access to hard-to-reach areas of a medical device, such as the posterior femoral condyles of a knee prosthesis. The tubular body 82 may additionally comprise at its distal tip a sponge as described in further detail with respect to Fig. 3C or a conical cap as described in further detail with respect to Fig. 3D. The tubular body 82 may have a length of between 20mm and 100mm.

Fig. 3B shows a fluid output 10 according to one or more embodiments for use with apparatus 1. The fluid output 10 comprises a tubular body 84 comprising one or more fluid conduits. The one or more fluid conduits provide a fluid paths for the fluid 14 from the fluid source 12 to the target area on the medical device 40. Optionally, the one or more fluid conduits are connected to the negative pressure source 60 so that the tubular body 84 also provides a fluid path 62 for the fluid extracted by the negative pressure source 60. Optionally, the second electrode 20 is provided at or proximal to the distal tip of the tubular body 84. The tubular body 84 may comprise a single orifice at its distal end for the fluid output so that the electrolytic fluid is output as a single jet of fluid, or the tubular body 84 may comprise a plurality of orifices at its distal end so that the electrolytic fluid is output as a shower or spray of fluid. The tubular body 84 is straight so that the longitudinal axis of the tubular body 84 remains unchanged along its length. The tubular body 84 may additionally comprise at its distal tip a sponge as described in further detail with respect to Fig. 3C or a conical cap as described in further detail with respect to Fig. 3D. The tubular body 84 may have a length of between 20mm and 100mm.

Fig. 3C shows a fluid output 10 according to one or more embodiments for use with apparatus 1. The fluid output 10 comprises a tubular body 85 comprising one or more fluid conduits. The one or more fluid conduits provide a fluid paths for the fluid 14 from the fluid source 12 to the target area on the medical device 40. Optionally, the one or more fluid conduits are connected to the negative pressure source 60 so that the tubular body 85 also provides a fluid path 62 for the fluid extracted by the negative pressure source 60. Optionally, the second electrode 20 is provided at or proximal to the distal tip of the tubular body 85. The tubular body 85 may comprise a single orifice at its distal end for the fluid output so that the electrolytic fluid is output as a single jet of fluid, or the tubular body 85 may comprise a plurality of orifices at its distal end so that the electrolytic fluid is output as a shower or spray of fluid. The fluid output further comprises a sponge 86 mounted to the distal end of the tubular body 85. The sponge 86 may be made of any deformable porous material such as polyester. The sponge 86 may comprise a mesh of conductive material and may therefore be connected to power source 22 and used as the electrode 20. The sponge 86 allows for the fluid 14 to be distributed across a wide surface area of the medical device 40 to be treated, and suspends the electrolytic fluid 14 in the sponge 86 to ensure that electrical current runs between the electrodes 50, 20. The tubular body 85 may have a length of between 20mm and 100mm.

Fig. 3D shows a fluid output 10 according to one or more embodiments for use with apparatus 1. The fluid output 10 comprises a tubular body 87 comprising one or more fluid conduits. The one or more fluid conduits provide a fluid path for the fluid 14 from the fluid source 12 to the target area on the medical device 40. Optionally, the one or more fluid conduits are connected to the negative pressure source 60 so that the tubular body 87 also provides a fluid path 62 for the fluid extracted by the negative pressure source 60. Optionally, the second electrode 20 is provided at or proximal to the distal tip of the tubular body 87. The tubular body 87 may comprise a single orifice at its distal end for the fluid output so that the electrolytic fluid is output as a single jet of fluid, or the tubular body 87 may comprise a plurality of orifices at its distal end so that the electrolytic fluid is output as a shower or spray of fluid. The tubular body 87 may comprise a conical cap 88 at its distal tip for interfacing with the surface of the medical device 40. The conical cap 88 may prevent leakage of the electrolytic fluid away from the target area of the medical device 40 when interfaced. The tubular body 87 may have a length of between 20mm and 100mm.

Fig. 3E shows a fluid output 10 according to one or more embodiments for use with apparatus 1. Fig. 3F shows a cross-sectional view of the fluid output 10 shown in Fig. 3E when taken along line A-A. The fluid output 10 comprises a tubular body 89 comprising one or more fluid conduits. The one or more fluid conduits provide a fluid path for the fluid 14 from the fluid source 12 to the target area on the medical device 40. The tubular body 89 may comprise a single orifice at its distal end for the fluid output so that the electrolytic fluid is output as a single jet of fluid, or the tubular body 89 may comprise a plurality of orifices at its distal end so that the electrolytic fluid is output as a shower or spray of fluid. Optionally, the one or more fluid conduits are connected to the negative pressure source 60 so that the tubular body 89 also provides a fluid path 62 for the fluid extracted by the negative pressure source 60. The second electrode 20 is provided at or proximal to the distal tip of the tubular body 87. The second electrode 20 forms an annulus inside the tubular body 89 as shown in Fig. 3F. In some embodiments the second electrode 20 may form only a portion of an annulus. The fluid output 10 further comprises first electrode 50 located radially inwards of the second electrode 20. The first electrode optionally may be exposed from the distal tip of the tubular body 89 so that it will easily contact the medical device 40 to form the cathodic connection. In other embodiments, the first electrode 50 is formed as the annulus and the second electrode 20 is provided radially inwards of the first electrode 50. Accordingly, there may be provided a single detachable body which comprises all of the first electrode 50, the second electrode 20 and the fluid output 10. The fluid output 10 may additionally comprise one or more of the following features: a curved shape as described with respect to Fig. 3A; a sponge 86 as described with respect to Fig. 3C; and a conical cap 88 as described with respect to Fig. 3D. The tubular body 89 may have a length of between 20mm and 100mm.

It will be appreciated that in any of the embodiments described with reference to Figs. 3A to 3F, the fluid output 10 may instead comprise first electrode 50 in place of second electrode 20, and second electrode 20 may be provided on a separate element of the apparatus.

In some embodiments, the apparatus 1 may be suitable for minimally invasive, percutaneous treatment when the medical device 40 is situated in the body. In particular, electrodes 50 and 20 may comprise metallic needles configured to be injected into the body so that the first electrode 50 forms an electrical connection to the medical device 40 and the second electrode 20 is locatable proximal the target area of the medical device 40. The fluid output 10 may also comprise a needle for penetrating into the body and for injecting the electrolytic fluid at the site of the medical device. Finally, the negative pressure source 60 may also comprise a needle for penetrating into the body and extracting fluid from the site, of the negative pressure source 60 may use the fluid path of the fluid output 10 when the negative pressure source 60 and fluid output 10 operate alternately. Once the needles are correctly placed, electrolytic fluid may be injected to the site of the medical device 40 and the electrical pulses may be applied to the electrolytic fluid to remove bacteria from the surface of the medical device 40. The negative pressure source 60 may then be operated to extract the electrolytic fluid containing the removed bacteria, thereby removing bacteria from the body and treating the infection site.

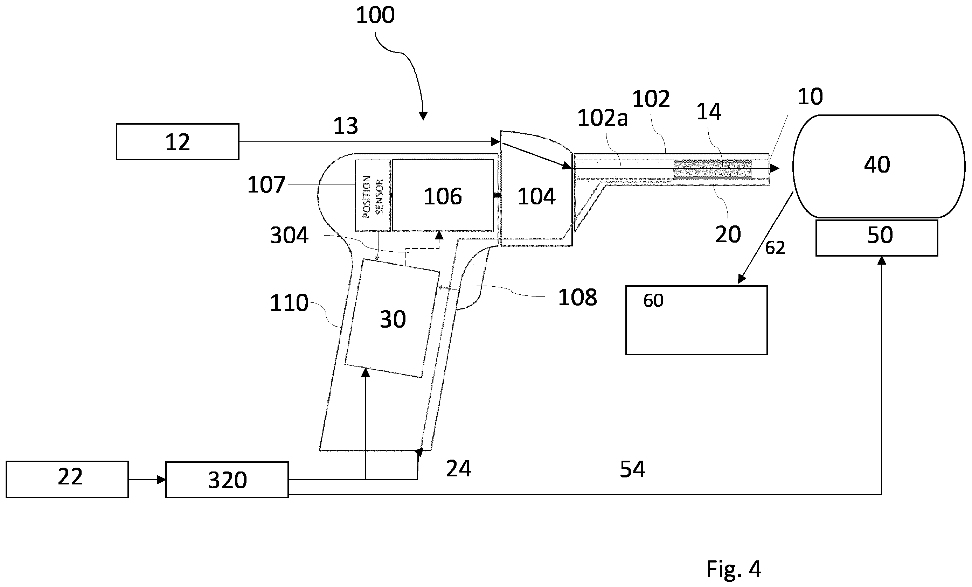

Fig. 4 shows a schematic diagram of an apparatus 100 for removal of biofilm or bacteria from a metallic medical device 40. As in the case of the apparatus 1 shown in Figs. 1A and 1B, the apparatus 100 comprises a fluid output 10, a first electrode 50 for electrically connecting to the metallic device 40, and a second electrode 20 and optionally a controller 30. The fluid output 10 is connectable to a fluid source 12 and is configured to supply an electrolytic fluid 14 to wash a target area of the medical device 40. The electrode 20 is located proximal to the fluid output 10 and connectable to an electric power source 22, optionally via controller 320. The apparatus 100 is configured to supply electrical power 24 between the electrodes 50, 20 such that electrolysis occurs at the target area. The electrical power source 22 is configured to provide intermittent electrical pulses between the first and second electrode 50, 20 such that electrolysis occurs at the surface of the metallic device 40, wherein the current applied in each electrical pulse is sufficient to cause bubbles to escape the surface of the metallic device 40 during the electrical pulse. The controller 30 is configured to control the supply of the fluid 14 from the source of fluid 12 to the fluid output 10 and the controller 320 is configured to control the supply of electrical power 24 to the electrodes 50, 20. In the illustrated embodiment the power source via controller 320 also powers the controller 30 and motor 106, whereas in other embodiments the controller 30 and motor 106 are powered by a separate power source. Furthermore, in some embodiments the controller 30 is configured to supply the fluid 14 intermittently.

In the embodiments shown in Fig. 4, the fluid output 10 and electrode 20 are removably connectable to the controller via a detachment mechanism. In the specific embodiment illustrated, the apparatus comprises a removable element 102 having a fluid conduit 102a, the fluid output 10 being located at the distal end of the conduit 102a. The removable element 102 may comprise a tubular body taking any of the forms described in relation to Figs. 3A to 3D and may further comprise a sponge or conical cap at the distal tip. The removable element 102 also comprises the electrode 20. The fluid output 10 and the electrode 20 are removably connected to a main body 110 via a detachment mechanism. For example, the removable element 102 is connectable to the main body 110 by a screw thread mechanism, a frictional fit or stab-type connection. It will be appreciated than any suitable detachment mechanism may be used which has a first configuration in which the removable element 102 and the main body 110 are mechanically connected, and a second configuration in which the removable element 102 and the main body 110 are disconnected. When the removable element 102 and the main body 110 are connected, the electrode 20 is connected to an electrical wire which extends to the proximal end of the removable element 102 and which forms an electrical connection with electrical wiring in the main body 110, which in turn is connectable to the power source 22. The electrical connection may be formed by an any suitable electrical connection, such as a plug and socket or pin connection. The electrode 20 and fluid output 10 being provided as removable from the controller 30 allows the electrode 20 and fluid output 10 to be replaced in between bacteria or biofilm removal operations, so that cross-contamination does not occur between medical devices. Whilst the removable element 102 includes both the fluid output 10 and electrode 20, in other embodiments the fluid output 10 and electrode 20 may be permanently connected to the controller 30 and the body 110, or only one of the fluid output 10 and the electrode may be connected to the controller 30 by a detachment mechanism. The fluid output 10 and the electrode 20 may instead be removably connected to the controller 30 by separate detachment mechanisms. The first electrode 50 may be removably connectable to the power source 22 or controller 320 by a similar detach mechanism so that the first electrode 50 can be replace with another electrode for washing different medical devices. In other embodiments the first electrode 50 may be permanently connected to the controller 320 or power source 22. The first electrode 50 may be connectable to the medical device by any suitable connecting mechanism, such as an electrical clip, or by an adhesive pad.

The apparatus 100 further comprises a pump 104 connectable between the fluid source 12 and the fluid output 10. The pump 104 may be any suitable pump such as a positive-displacement pump, impulse pump or rotodynamic pump. In preferred embodiments the pump is a peristaltic pump. In the illustrated embodiment, the pump 104 forms part of the removable element 102, and when the removable element 102 is connected to the body 110 via the detachment mechanism, the pump 104 forms a mechanical connection with an electric motor 106 for actuating the pump 104. In other embodiments, the pump 104 is hydraulically actuated and the apparatus comprises a source of hydraulic pressure for actuating the pump. The pump 104 is also configured to be connected to the fluid source 12 in order to receive the fluid 14 as an input to the pump. The pump 104 may be connected to the fluid source 12 independently of the body 110, as illustrated in Fig. 4. For example, the pump 104 may comprise a fluid input port configured to receive a fluid conduit connected to the fluid source 12. In other embodiments, the fluid source 12 may be connected to the pump 104 through a fluid path in the body 110. For example, the body 110 may comprise a fluid input port configured to receive a fluid conduit connected to the fluid source 12, and the pump 104 may be configured to receive the fluid 14 via a fluid path through the body 110. In some embodiments, the pump 104 is provided in or on the body 110. In such embodiments, the body 110 is configured to be connected to both the power source 22 and the fluid source 12, and the removable element 102 when connected to the body 110 forms a fluidic connection between the output of the pump 104 and the fluid output 10 and an electric connection between the power source 22 and the electrode 20 via the body 110.

In the embodiment shown in Fig. 4, the controller 30 is configured to control operation of the motor 106 and thus the pump 104. Thus, the controller controls the flow of fluid 14 to the fluid output 10 by controlling the motor 106.

As shown in Fig. 4, body 110 may be shaped ergonomically such that it can be gripped in the hand of a user. The apparatus 100 may further comprise a user actuated element 108, such as a trigger, for actuating the supply of the electrolytic fluid to the fluid output 10. In embodiments where the controller 30 also controls the supply of electric power to the electrodes 50, 20, the user actuated element 108 (or a separate user actuated element) may also be provided for actuating the supply of electric power to the electrodes 50, 20. The user actuated element 108 is connected to the controller 30. When the user actuated element 108 is set to a first configuration by a user, the controller 30 is configured to actuate the motor 106. When the user actuated element 108 is set to a second configuration by a user, the controller 30 is configured to stop the motor 106. In the illustrated embodiment the user actuated element 108 is a spring-loaded trigger, wherein the first configuration is achieved by pulling the trigger, and the second configuration is achieved by releasing the trigger. The user actuated element may be connected to, for example, a switch for switching on and off the motor, or the user actuated element may be configured to send a start or stop signal to the controller 30 in the first and second configurations. In other embodiments, for example, the user actuated element 108 may comprise one or more buttons or levers, or may comprise a guided user interface on a touchscreen.

The apparatus 100 may further comprise a negative pressure source 60 configured to remove fluid from the target area. The negative pressure source 60 may be any suitable negative pressure source such as a vacuum pump or similar and may be provided with the main body 110 or separately to the main body 110. The negative pressure may be transmitted from the source 62 to the target area via flexible tubing positionable at the target area of the medical device 40 and forming a fluid path 62. The negative pressure source 60 may be powered by any suitable internal or external power source, and may be powered by power source 22. The negative pressure source 60 may be configured to operate intermittently so that excess electrolytic fluid is removed from the surface of the medical device 40 intermittently. The negative pressure source 60 may be controlled by controller 30 via any suitable control connection (not shown). The controller 30 may be configured to synchronize the intermittent supply of fluid 14 with the intermittent operation of the negative pressure source 60 so that the intermittent negative pressure source 60 operates a predetermined period of time after fluid output 10 stops supplying fluid 14 to the medical device 40. The negative pressure source 60 may output the received fluid to a storage reservoir (not shown). The negative pressure source 60 may be comprised in the main body 110 and powered by a motor in the main body 110 controlled by controller 30, as in the case of pump 104. The removable element 102 may comprise a second fluid conduit (not shown) extending through the removable element 102 from the fluid output 10 to the negative pressure source 60, when the removable element 102 is attached to the main body 110.

For the apparatus 100, the surface area of the electrode 20 for determining the current density at the electrode 20 is the longitudinal cross-sectional area of the electrode 20 at the distal end of the removable element 102.

Fig. 5A shows a controller 320 for controlling electric power supplied to the electrode 20. The controller 320 is powered by power source 22 (which may be external or internal to controller 320) and may comprise a user interface 321, such as a touchscreen, for selecting parameters of the power supply such as voltage, current, pulse rate and duration. The controller 320 comprises an output 326. For example, the output 326 is connectable to a power cable which is electrically connected to electrode 20. For example, in the embodiment shown in Fig. 4, the body 110 is connectable to the output 326 via a power cable which provides the electric connection to the electrode 20 (and optionally power to the controller 30 and motor 106). The controller 320 may further comprise an electrical connection 328 for the first electrode 50 or the first electrode 50 may be permanently connected to the controller 320. The controller 320 is configured to provide DC electrical pulses between the electrodes 50, 20 so that electrolysis is able to occur.

Fig. 5B shows another controller 330 for controlling electric power supplied to the electrode 20. The controller 320 is powered by power source 22 (which may be external or internal to controller 320) and may comprise a user interface 321, such as a touchscreen, for selecting parameters of the power supply such as voltage, current, pulse rate and duration. The controller 330 comprises an output 326. For example, the output 326 is connectable to a power cable which is electrically connected to second electrode 20. The controller 330 may further comprise an electrical connection 328 for the first electrode 50 or the first electrode 50 may be permanently connected to the controller 330. The controller 330 is configured to provide DC electrical pulses between the electrodes 50, 20 so that electrolysis is able to occur. The controller 330 additionally comprises a pump 104 incorporated into the controller 330 so that the controller 330 also provides the fluid to the fluid output 10 (the pump 104 controller 330 may be connectable to any suitable fluid source via a fluid connection of the controller 330, or the controller 330 may further comprise the fluid source). The pump may be for example a peristaltic pump so that the pump parts are not exposed to the fluid. The pump 104 may comprise an output connector 329 for connecting to a proximal end of the fluid output 10.

Fig. 5C shows a fluid output 10 that may be used in conjunction with controller 330. As the pump is incorporated into the controller 330, the fluid output 10 is free from any moving parts. The fluid output 10 of Fig. 5C comprises tubular body 350 comprising one or more fluid conduits from a proximal end of the tube 350 to a distal end of the tube 50. The tubular body 350 may, for example, be any of the tubular bodies described with respect to Figs. 3A to 3F. The fluid output 10 further comprises a connector 351 at a proximal end of the tubular body 350. The connector 351 is configured to mate with the output connector 329 to form a fluidic connection between the pump 104 and the one or more fluid conduits of the tubular body 350 so that the pump 104 provides fluid to the distal end of the tubular body 350 through the one or more fluid conduits. The connector 351 may form any type of fluidic connection with the output connector 329, such as a frictional fit, a screw thread fit or similar. The fluid output 10 may further comprise leads 352 and 353 for connecting to electrical outputs 326 and 328 to electrically connect electrodes 20 and/or 50 to the controller 330 when the electrodes 20 and/or 50 are provided with the tubular body 350.

In any of the embodiments disclosed herein, the fluid 14 may be any electrolyte solution (for example a saline solution such as physiological saline) and the apparatus is configured to apply DC electrical pulses between the electrodes 50, 20. The second electrode 20 may be selected as the cathode or the anode and the first electrode 50 is connectable to the medical device. Preferably, the first electrode 50 is the cathode so that oxidation or electro-corrosion of the medical device 40 does not occur. The DC electrical power applied between the electrodes 50, 20 causes electrolysis to occur at the target area. The bubbles formed at the target area of the medical device lift up bacteria from the surface of the medical device, reducing adhesion of the bacteria to the medical device and removing biofilms from its surface. In some embodiments, the fluid output is configured to provide the fluid intermittently, which provides intermittent washing to the medical device 40 to prevent the build-up of bacteria in the electrolytic fluid at the surface. That is to say, the fluid output 10 provides the electrolyte solution for allowing the electrolysis at the target area, and may further wash away the detached bacteria by the intermittently supplied fluid at the target area. The electrolyte solution fluid is comprised of a solvent and electrolytes. The solvent may comprise, for example, water. The electrolytes may comprise, for example, salt ions and/or silver ions. When the electrolyte solution contains silver ions, an antibacterial effect is observed without the need to us a sacrificial electrode such as a silver electrode. The electrolyte solution may further comprise one or more of a disinfecting agent (such as hypochlorous acid), an antibacterial agent (such as dissolved silver, gold or copper ions), or an antibiotic therapeutic composition (the antibiotic therapeutic composition may comprise any suitable antibiotic composition known in the art, including but not limited to one or more of vancomycin, gentamicin and clindamycin). Saline solution is advantageously biocompatible. It is noted that the cleaning effect from the electrolysis and periodic washing at the target area arises from the removal of bacteria from the surface by the bubbles formed during electrolysis, and the washing away of the removed bacteria by the periodic washing. It has been observed that this process greatly shortens the cleaning time of the medical device when compared to the process of indirectly killing bacteria using the toxic chemicals produced by the process of electrolysis.

The electric DC power is applied intermittently. When the fluid is output intermittently, at least one cycle of DC current may be provided to the electrode for each cycle of fluid provided to the fluid output. The provision of at least one cycle of DC current for each cycle of fluid output ensures that bacteria is lifted from the surface of the medical device before the next fluid wash occurs. Preferably, the DC current and the fluid are provided intermittently so that the electrolysis occurs in between washes of the medical device by the fluid. Fig. 6 shows a graph of the fluid pressure 602 applied at the fluid output over time compared with the current amplitude 604 applied at the electrode 20. For example, the pressure profile 602 may be provided by the pump 104 controlled by controller 30 and the current profile 604 may be provided by the controller 320. The pressure profile 602 comprises a series of pulses which provide washing of the medical device at a desired pressure. The current profile 604 provides intermittent periods of constant current subsequent to the fluid pressure pulse. The alternate fluid wash and electrolysis caused by the direct current provides efficient cleaning to the medical device.

In a preferred embodiment, the second electrode 20 is a sacrificial anode comprising a sacrificial metal. As disclosed herein, a sacrificial electrode may be defined as an electrode that disintegrates over time when a current is applied to the electrode in an electrolytic solution. The sacrificial electrode may be selected to release particular ions into the electrolytic solution which have antibacterial properties. Examples of suitable materials include silver and copper.

In a further preferred embodiment, the sacrificial anode comprises silver (Ag) so that the oxidation process at the anode from the electrolysis releases silver ions to the target area of the device 40. The silver ions increase the antimicrobial properties of the fluid exposed to the medical device. Thus, the potency of the biofilm or bacterial removal process is increased.

In other embodiments, the second electrode 20 is free from sacrificial metals and comprises a non-corrodible metal, so that little to no metal ions are released into the electrolytic solution during electrolysis. This allows the electrolytic solution to be retrieved after electrolysis which contains bacteria from the biofilm. The bacteria may be isolated from the electrolytic solution and cultured so that the bacteria can be identified in order to guide treatment of the infection, such as the effective course of antibiotics to administer to the patient. Examples of suitable materials include platinum.

In any of the embodiments disclosed herein, the first electrode 50 may comprise one or more non-corrodible metals such as cobalt-chromium and titanium.

In any of the embodiments disclosed herein, the apparatus may be configured to provide a fluid flow of electrolytic fluid to the fluid output of between 0.5 and 2 litres per minute. The flow may be continuous or intermittent with a pulse rate of between 0-100Hz, and more preferably 0-10Hz.

In any of the embodiments disclosed herein, the apparatus may be configured to provide DC pulses having a duration between 0.05 and 0.2 seconds, at a frequency of between 0-100Hz, and more preferably 0-10Hz. The apparatus may be configured to provide a current of between 0 and 1.5A.

In any of the embodiments disclosed herein, the fluid output 10 may be removably connectable to the controller 30 via a detachment mechanism as disclosed in other embodiments. The fluid output 10 and electrode 20 may be provided on the same detachable body.

Any of the embodiments disclosed herein may comprise a pump situated between the fluid source 12 and the fluid output 10, wherein the controller is configured to control the pump to provide fluid to the fluid output 10 as a predetermined pressure.

Any of the embodiments disclosed herein may comprise one or more user interfaces to select the operating parameters of the fluid supply and the electric supply.

In any of the embodiments disclosed herein, the fluid output 10 and first and/or second electrode 50, 20 may be connectable to a handheld portion, wherein the handheld portion comprises a user actuated element for actuating the supply of fluid and/or electrical power.

Any of the apparatuses disclosed herein may be used to implement the following method of removing biofilm from, or reducing bacterial adhesion to, a metallic medical device internal or external to the human or animal body:
- connecting a first electrode to a surface of the medical device (for example electrode 50);
- exposing a target area on the surface of the medical device to an electrolytic fluid (for example using fluid output 10);
- placing a second electrode proximal to the target area (for example electrode 20);
- providing electrical power between the first and second electrode such that electrolysis occurs in the electrolytic fluid (for example using power source 22);

- wherein the electrical power comprises a plurality of electrical pulses, wherein the current applied in each electrical pulse is sufficient to cause bubbles to escape the surface of the medical device.

It is noted that the method is not limited to being applied by the above-disclosed devices. For example, the method could be implemented by submerging the medical device in a reservoir or container of the electrolytic fluid, connecting a first electrode to a surface of the submerged medical device, placing a second electrode proximal to the target area of the medical device, and providing the required electrical pulses between the electrodes such that the electrolysis occurs on the surface of the medical device. It is noted that in embodiments where the medical device is submerged in a reservoir of the electrolytic fluid in this manner, there may further comprise the step of washing the medical device with the electrolytic fluid, for example by using a fluid output as disclosed herein for washing the device with fluid.

The fluid may be any electrolyte solution disclosed herein.

Exposing the target area to an electrolytic fluid may comprise washing the metallic device intermittently with the electrolytic fluid. The washing and electrical pulses may be applied alternately (e.g. as shown in Fig. 6). The fluid and electrical pulses may be applied according to any of the embodiments disclosed herein.

The second electrode may be any sacrificial anode disclosed herein, and preferably the anode is comprised of silver.

In some embodiments, the second electrode is free from sacrificial metals and comprises a non-corrodible metal, so that little to no metal ions are released into the electrolytic solution during electrolysis. This allows the electrolytic solution to be retrieved after electrolysis which contains bacteria from the biofilm. The method may then comprise isolating bacteria from the electrolytic fluid after the electrical power is provided. The bacteria may be isolated from the electrolytic solution and cultured so that the bacteria can be identified in order to guide treatment of the infection, such as the effective course of antibiotics to administer to the patient.

The above methods may also be applied to a medical device in vitro. Furthermore, the above methods may also be applied to a target area on or in the human or animal body directly.

### EXAMPLE 1

Fig. 7A shows experimental data of the reduction in bacteria on a metallic device in a first example.

In the first example, nine cathodic bases (three grouped "CH1", three grouped "CH2", three grouped "CH3") comprising plates made of cobalt-chrome (CoCr) and nine corresponding anodic plates also made of cobalt-chrome (CoCr) were used. The plates were discs having a surface area of 1.54cm². Before use, the plates were sterilised with UV light for 30 minutes. The cathodic bases were submerged in respective wells of 3 mL. The wells were filled with a culture medium of TSB (Trypticase soy broth) supplemented with 0.25% concentration glucose. A 1/100 dilution of a Staphylococcus epidermidis strain was used in the culture medium. The plates were incubated for 24 hours at 37 °C. After 24 hours of incubation, the culture medium was removed from the wells and each well was washed three times with sterile phosphate-buffered saline (PBS 1X). Each well was then filled with 3 mL of saline solution such that the saline electrically connected the respective anodes and cathodes. A plurality of control plates were also incubated in the same manner. The following series of electrical pulses were applied between the respective anodes and cathodes so that electrolysis occurred in the wells and bubbles escaped the surface of the cathodes (the control plates were not exposed to electrical current):

| **Well** | **Voltage (V)** | **Pulse duration (s)** | **Time between pulses (s)** | **Total number of pulses** |
|---|---|---|---|---|
| CH1 | 15 | 0.1 | 0.5 | 100 |
| CH2 | 15 | 0.1 | 0.5 | 150 |
| CH3 | 15 | 0.1 | 0.5 | 200 |

Following the series of electrical pulses, the saline solution was removed from the wells (this removed saline solution was used to measure the number of bacteria in the planktonic state after the pulses as explained below), and each well was washed again three times with sterile PBS 1X (including the control wells). The biofilm adhering to the cathode was removed with a sterile spatula and then homogenised in 900 µL saline solution. One hundred microlitres of the different serial dilutions were seeded on Müller Hinton (MH) agar plates for CFU/mL counting. Data were transformed to CFU/cm² according to the formula: $CFU / {cm}^{2} = \frac{\left(CFU/mL∗dilution factor\right)}{\left(area of the disc\right)}$

Furthermore, and following the previous methodology of dilutions, the number of bacteria in the planktonic state (i.e. not adhered to the surface of the cathode) following the series of electrical pulses was also measured by measuring the amount of bacteria contained in the saline solution removed from the wells directly after the electrical pulses (or at the corresponding time for the control experiment). The data was transformed to CFU/cm² according to the formula above.

Fig. 7A shows the average CFU/cm² values for the removed biofilms from the control CH1, CH2 and CH3 groups respectively (bars Control BF, CH1-BF, CH2-BF and CH3-BF), and the CFU/cm² values for the bacteria in the planktonic state from the control, CH1, CH2 and CH3 groups respectively ("Control planktonic" and C_plan_DP"). It can be seen that a significant (several orders of magnitude) removal of biofilm occurred for all groups compared to the control wells ("Control BF"), with a p value of less than 0.0001. Similarly, it can be seen that a significant (several orders of magnitude) removal of bacteria in the planktonic state occurred for all groups.

### EXAMPLE 2

Fig. 7B shows experimental data of the reduction in bacteria on a metallic device in a second example.

In the second example, nine cathodic bases (three grouped "CH1", three grouped "CH2", three grouped "CH3") comprising plates made of cobalt-chrome (CoCr) and nine corresponding anodic plates made of silver (Ag) were used. The plates were discs having a surface area of 1.54cm². Before use, the plates were sterilised with UV light for 30 minutes. The cathodic bases were submerged in respective wells of 3 mL. The wells were filled with a culture medium of TSB (Trypticase soy broth) supplemented with 0.25% concentration glucose. A 1/100 dilution of a Staphylococcus epidermidis strain was used in the culture medium. The plates were incubated for 24 hours at 37 °C. After 24 hours of incubation, the culture medium was removed from the wells and each well was washed three times with sterile phosphate-buffered saline (PBS 1X). Each well was then filled with 3 mL of saline solution such that the saline electrically connected the respective anodes and cathodes. A plurality of control plates were also incubated in the same manner. The following series of electrical pulses were applied between the respective anodes and cathodes so that electrolysis occurred in the wells and bubbles escaped the surface of the cathodes (the control plates were not exposed to electrical current):

| **Well** | **Voltage (V)** | **Pulse duration (s)** | **Time between pulses (s)** | **Total number of pulses** |
|---|---|---|---|---|
| CH1 | 15 | 0.1 | 0.5 | 25 |
| CH2 | 15 | 0.1 | 0.5 | 50 |
| CH3 | 15 | 0.1 | 0.5 | 100 |

Following the series of electrical pulses, the saline solution was removed from the wells (this removed saline solution was used to measure the number of bacteria in the planktonic state after the pulses as explained below), and each well was washed again three times with sterile PBS 1X (including the control wells). The biofilm adhering to the cathode was removed with a sterile spatula and then homogenised in 900 µL saline solution. One hundred microlitres of the different serial dilutions were seeded on Müller Hinton (MH) agar plates for CFU/mL counting. Data were transformed to CFU/cm² according to the formula: $CFU / {cm}^{2} = \frac{\left(CFU/mL∗dilution factor\right)}{\left(area of the disc\right)}$

Furthermore, and following the previous methodology of dilutions, the number of bacteria in the planktonic state (i.e. not adhered to the surface of the cathode) following the series of electrical pulses was also measured by measuring the amount of bacteria contained in the saline solution removed from the wells directly after the electrical pulses (or at the corresponding time for the control experiment). The data was transformed to CFU/cm² according to the formula above.

Fig. 7B shows the average CFU/cm² values for the removed biofilms from the control, CH1, CH2 and CH3 groups respectively (bars Control BF CH1-BF, CH2-BF and CH3-BF), and the CFU/cm² values for the bacteria in the planktonic state from the control, CH1, CH2 and CH3 groups respectively ("Control planktonic" and C_plan_DP"). It can be seen that a significant (several orders of magnitude) removal of biofilm occurred for all groups compared to the control wells ("Control BF"), with a p value of less than 0.0055. Similarly, it can be seen that a significant (several orders of magnitude) removal of bacteria in the planktonic state occurred for all groups. The reduction of biofilm and planktonic bacteria increases significantly with a greater number of pulses.

It is noted that the high capacity of the electrical pulses to detach the biofilm is observed with only 25 electrical pulses of 0.1 seconds duration every 0.5 second, achieving a reduction by eight orders of magnitude with respect to the control over a very short time of less than 30 seconds.

### EXAMPLE 3

Fig. 7C shows experimental data of the reduction in bacteria on a metallic device in a third example.

In the third example, nine cathodic bases (three grouped "CH1", three grouped "CH2", three grouped "CH3") comprising plates made of cobalt-chrome (CoCr) and nine corresponding anodic plates made of silver (Ag) were used. The plates were discs having a surface area of 1.54cm². Before use, the plates were sterilised with UV light for 30 minutes. The cathodic bases were submerged in respective wells of 3 mL. The wells were filled with a culture medium of TSB (Trypticase soy broth) supplemented with 0.25% concentration glucose. A 1/100 dilution of a Staphylococcus epidermidis strain was used in the culture medium. The plates were incubated for 24 hours at 37 °C. After 24 hours of incubation, the culture medium was removed from the wells and each well was washed three times with sterile phosphate-buffered saline (PBS 1X). Each well was then filled with 3 mL of saline solution such that the saline electrically connected the respective anodes and cathodes. A plurality of control plates were also incubated in the same manner. Electrical pulses were applied between the respective anodes and cathodes of groups CH1 and CH3 so that electrolysis occurred in the wells and bubbles escaped the surface of the cathodes. Following the electrical pulses, the saline solution was removed from the wells in group CH1 and each well in group CH1 was washed again three times with sterile PBS 1X (including the control wells). The biofilm adhering to the cathodes of CH1 were each removed with a sterile spatula and then homogenised in 900 µL saline solution. One hundred microlitres of the different serial dilutions were seeded on Müller Hinton (MH) agar plates for CFU/mL counting. Data were transformed to CFU/cm² according to the formula: $CFU / {cm}^{2} = \frac{\left(CFU/mL∗dilution factor\right)}{\left(area of the disc\right)}$

The cathodic plates in groups CH2 and CH3 were removed from the wells and washed with a pressured jet of saline solution, making sure to avoid cross-contamination of the plates by splashes. Once the pressured washing was finished the biofilm adhering to the cathodes of CH2 and CH3 were each removed with a sterile spatula and then homogenised in 900 µL saline solution. One hundred microlitres of the different serial dilutions were seeded on Müller Hinton (MH) agar plates for CFU/mL counting. Data were transformed to CFU/cm² according to the formula above.

Accordingly, the CH1 plates were exposed only to electrolysis, the CH2 plates were exposed to only washing using a pressured jet of fluid, and the CH3 plates were exposed to both electrolysis and washing using the pressured jet. The parameters of the electrolysis and washing were as follows:

| **Well** | **Voltage (V)** | **Pulse duration (s)** | **Time between pulses (s)** | **Total number of pulses** | **Duration of wash (s)** |
|---|---|---|---|---|---|
| CH1 | 15 | 0.1 | 0.5 | 25 | - |
| CH2 | - | - | - | - | 25 |
| CH3 | 15 | 0.1 | 0.5 | 25 | 25 |

Fig. 7C shows the average CFU/cm² values for the removed biofilms from the control, CH1, CH2 and CH3 groups respectively (bars Control BF, Pulse 25, Pistol and Pulse 25+Pistole), and the CFU/cm² values for the bacteria in the planktonic state from the control ("Control planktonic"). The number of bacteria in the planktonic state for the control was measured in the same manners as for Examples 1 and 2. It can be seen that the application of only 25 electrical pulses generates a reduction in biofilm by about 7.8 orders of magnitude as compared to the control, compared to a reduction of about 4.7 orders of magnitude when only washing is used. The combination of electrical pulses and washing resulted in a reduction in biofilm of about 9.9 orders of magnitude.

### EXAMPLE 4

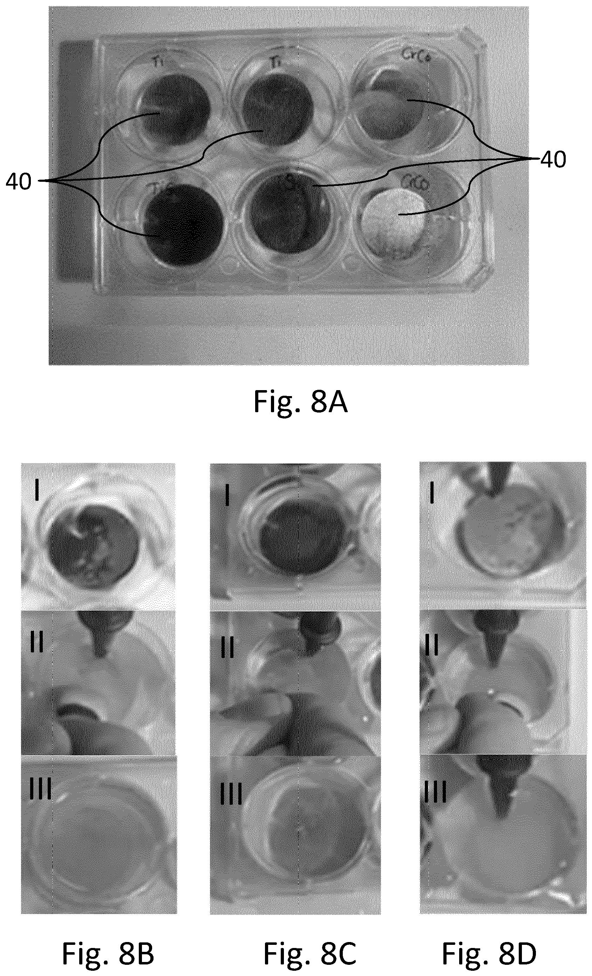

Fig. 8A shows a set of wells wherein different medical implants (40) of Ti, Ti sintered and Cr-Co have been cultured with a certain biofilm. Figs. 8B-8D shows the biofilm elimination on a Ti, Ti sintered and Cr-Co medical implant (40) during an in-vitro method according to the present operation, respectively.

In the fourth example, a biofilm of E. Coli was grown and developed in 1.4 cm wide plates of Ti, Ti sintered and Cr-Co for 72h to form a mature biofilm, that was dyed with violet crystal such that the biofilm can be easily identified as shown in stage I of Fig. 8B-8D by their darker colour. Subsequently, to increase its adherence it was dehydrated for another 72 hours.

Once this was done, it was tried to detach with physiological serum, this being impossible.

Subsequently, a pulse of 1A (2 pulses per second, 0.25s duration each pulse) was applied (see stage II of Fig. 8B-8D), managing to detach the entire biofilm in less than 10 seconds of application as shown in stage III of Fig. 8B-8D, wherein the dyed biofilm has been completely removed from the medical implant (40). The 1.4 cm wide disc plates, had a target area of 1.54 cm² , which means that a current density of 0.65A /cm² was applied.

This in vitro experiment shows that the in vitro method of removing biofilm according to the present invention can effectively remove the biofilm on a medical implant in a short period of time.

### EXAMPLE 5

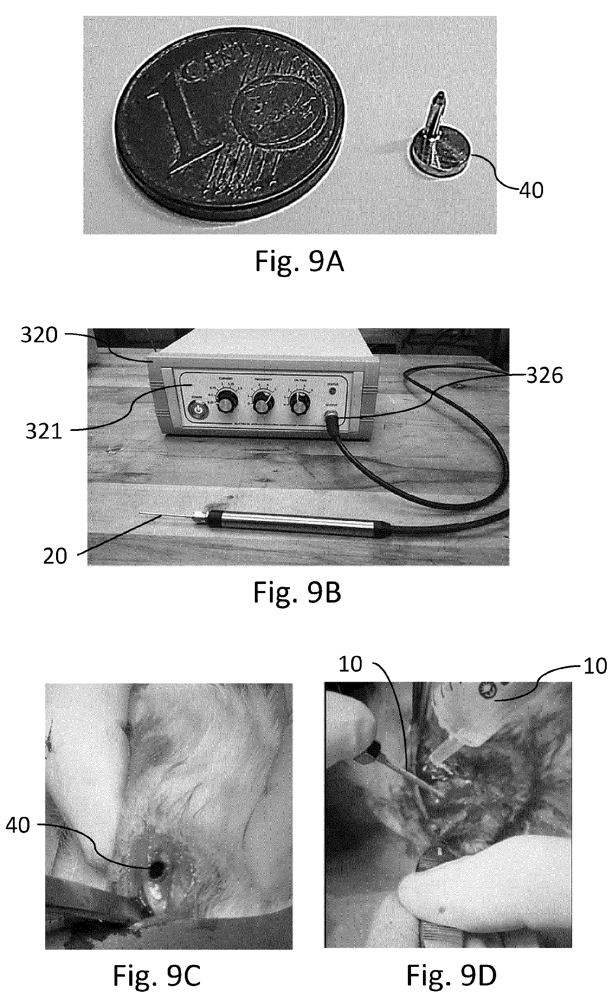

Fig. 9A shows a medical implant (40) for a rabbit knee made of Cr-Co of just 3mm diameter. Fig 9B shows a part of the apparatus used for removal of biofilm from a metallic medical device, comprising: a controller 320, and a first electrode 20. The controller 320 comprises a user interface 321 and an output 326 connectable to a power cable which is electrically connected to electrode 20.

In the fifth example an in vivo test was performed. Two New Zealand White rabbits were implanted with a specially designed Cr-Co prosthesis (40) in both knees as shown in Fig. 9C, under standard asepsis measures and antibiotic prophylaxis with cefazolin 10mg / kg in a single dose. Prior to implantation of the prostheses, the prostheses were immersed in a 0.3 McFarland inoculum of S. aureus ATCC 25923 for 30 seconds.

In the first animal (control) the joint was closed directly after washing with saline solution. In the second animal (case) treatment was administered on both prostheses with pulses of electric current (1A, 2 pulses per second) with a time gap of 30% for 40 seconds, washing with saline and closure of the joint as shown in Fig. 9D. The 3 mm prostheses, had a target area of 0.07 cm² , which means that a current density of 14.29A /cm² was applied.

After 72 hours both animals were reoperated to take samples of tissues, bone and explant of the prostheses to perform cultures. In all samples quantitative cultures were previously vortexed and sonicated in one millilitre of BHI. Absence of contamination by identification by MALDI-TOF MS (VITEK-MS) and automated antibiogram of isolated colonies (VITEK-2) was confirmed.

In the control animal, treated with a dose of cefazolin isolates S. aureus in all samples studied, the bacterial count expressed as log10 (ufc/cm²) in the right leg prosthesis was 9.38 and in the left leg 8.86. The bacterial count expressed as log10 (ufc/mL) in bone and periprosthetic tissue biopsies was 2.70 and 2.72 in the right leg and 3.24 and 3.87 in the left leg respectively. In the animal in which an electric field was applied to the implant after its placement in addition to a dose of cefazolin, all samples (prosthesis, bone and periprosthetic tissue biopsies) were negative, not being able to isolate the strain of S aureus inoculated after incubation of the sonicated broth for 14 days. The results can be observed in the following table:

| | Control | | Case | |
|---|---|---|---|---|
| Knee | Right | Left | Right | Left |
| Prosthesis (ufc/cm²) | 9,38 | 8,86 | - | - |
| Bone (ufc/mL) | 2,70 | 3,24 | - | - |
| Periprosthetic tissue (ufc/mL) | 2,72 | 3,87 | - | - |

This in vivo model suggests the potential efficacy of the application of an electric field on a prosthetic implant in combination with cefazolin in preventing the development of IPA after exposure of the implant to an inoculum of S. aureus ATCC 25923.

### EXAMPLE 6

In the sixth example another in vivo test was performed. Two New Zealand White rabbits were implanted with a specially designed Cr-Co prosthesis, under standard asepsis measures and antibiotic prophylaxis with cefazolin 10mg / kg in a single dose.

Then, 0,5-1 mL of serum comprising an inoculum of S. aureus ATCC (10⁵ ufc/ml) was provided and the rabbits were stalled for 4 days to let the infection progress.

Once the infection had progressed for 4 days, the first rabbit (control) and the second rabbit (case) were treated differently. The control rabbit had an extensive surgical cleaning and washing with abundant physiological serum for 1 minute, representing a debridement, antibiotics and implant retention (DAIR) typical intervention. The case rabbit had an extensive surgical cleaning and washing with abundant physiological serum and electrical current pulses (1A, 2 pulses per second with a duty cycle of 30% ) for 1 minute.

After the cleaning procedures, both control and case rabbits were monitored for 4 days, keeping the cefazolin doses to prevent sepsis and the progression of the infection to soft tissues.

Finally, the prosthesis were explanted in sterile conditions, further taking samples of the joint fluid, periarticular tissues, bone, and blood cultures.

Results show that in the control animal the bacterial count expressed as log10 (ufc/mL) in a 1/10 dilution of each prosthesis (data without accounting for the prosthesis surface) was significantly greater than in the case animal, as shown in the following table:

| | Control | | Case | |
|---|---|---|---|---|
| Knee | Right | Left | Right | Left |
| Prosthesis (ufc/mL) in a 1/10 dilution | 45 | 41 | 8 | 0 |

Results shown that electric fields applied according to one or more embodiments of the present invention are very effective for the treatment of articulation prosthetic infections improving results compared to when only DAIR (debridement, antibiotics and implant retention) is performed. The bacterial count on the right knee of the case rabbit can be explained by the recolonization from the bacteria present in the periarticular soft tissues. However, the count was much lower than in prostheses of the control rabbit treated only with DAIR (without electric fields).

### EXAMPLE 7

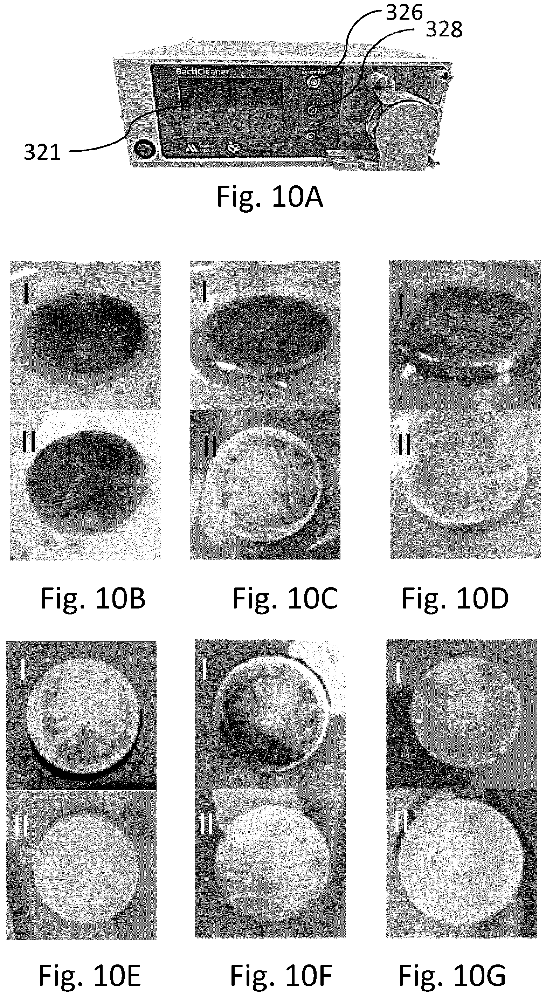

Fig 10A shows a part of the apparatus used for removal of biofilm from a metallic medical device according to one or more embodiments of the present invention, comprising: a controller 320, and a first electrode 20. The controller 320 comprises a user interface 321 and an output 326 connectable to a power cable configured to electrically connect to electrode 20 and an output 328 connectable to a power cable configured to electrically connect to reference electrode 50.

In the seventh example another in vitro test was performed. Figs. 10B-10G shows a set of wells wherein different medical implants (40) of Ti, Ti sintered and Cr-Co have been cultured with a certain biofilm. Figs. 10B-10D shows the biofilm state before and after a biofilm removal procedure based on pulsed fluids was performed on each of the Ti, Ti sintered and Cr-Co medical implants (40) respectively. Figs. 10E- 10G shows the biofilm state before and after a biofilm removal procedure according to the present invention was performed on each of the Ti, Ti sintered and Cr-Co medical implants (40) respectively.

A biofilm of E. Coli was grown and developed in plates of Ti, Ti sintered and Cr-Co for 72h to form a mature biofilm, that was dyed with violet crystal such that the biofilm can be easily identified as shown in stage I of Fig. 10B-10G by their darker colour (stage I). Subsequently, to increase its adherence it was dehydrated for another 72 hours.

First, three implants (of Ti, Ti sintered and Cr-Co) were treated using an electrolytic fluid in a pulsed manner according to the state of the art (Figs. 10B-10D). The electrolytic fluid was provided using a commercial fluid pistol at high and low washing flow.

Then, another three implants (of Ti, Ti sintered and Cr-Co) were treated using the apparatus according to one or more embodiments of the present invention of Fig. 10A, particularly, using a sponge on the fluid output distal end (Figs. 10E-10G). The electrical current pulses provided had 1A, 2 pulses per second with a duty cycle of 50% and the electrolytic fluid was provided at its minimum flow.

As shown in stage II of Figs. 10B-10G, it was demonstrated that in the implants treated only with pulsed electrolytic fluid, it was not possible to unhook the biofilm with either low or high washing flow. On the other hand, using a device and a method according to the present invention, the biofilm was totally removed in less than 5 seconds of application (for each implant).

All of the above are fully within the scope of the present disclosure, and are considered to form the basis for alternative embodiments in which one or more combinations of the above described features are applied, without limitation to the specific combination disclosed above. In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such modifications or adaptations fall within the scope of the present disclosure.

## Claims

1. An *in vitro* method of removing biofilm from, or reducing bacterial adhesion to, a metallic medical device external to the human or animal body, comprising:
connecting a first electrode (20) to a surface of the medical device;
exposing a target area on the surface of the medical device to an electrolytic fluid;
placing a second electrode (50) proximal to the target area;
providing electrical power between the first and second electrode such that electrolysis occurs in the electrolytic fluid;
wherein the electrical power comprises a plurality of electrical pulses, wherein the potential difference between the first and second electrode is at least 1V;
**characterized in that** the current applied in each electrical pulse is up to 2A, and wherein each electrical pulse has a duration of less than 1 second.

2. The method according to claim 1, wherein the step of providing electrical power between the first and second electrodes comprises providing a potential difference between 1V and 50V, preferably between 10V and 30V, and the step of providing electrical power between the first and second electrodes further comprises providing a current between the first and second electrodes between 0.5A and 1A.

3. The method according to claim 1 or 2, wherein each electrical pulse has a duration between 0.05 and 0.2 seconds.

4. The method according to any one of claims 1 to 3, wherein the second electrode is an anode comprising a sacrificial metal, preferably comprised of silver, and/or wherein the electrolytic fluid comprises one or more of a disinfecting agent, antibacterial agent or antibiotic agent; or
wherein the second electrode is an anode comprising a non-corrodible metal and free from sacrificial metals, wherein the method further comprises isolating bacteria from the electrolytic fluid after the electrical power is provided.

5. An apparatus for removal of biofilm from a metallic medical device, comprising:
a fluid output connectable to a source of electrolytic fluid, the fluid output for supplying the electrolytic fluid to wash a target area of the medical device;
a first electrode (20) for electrically connecting to the medical device;
a second electrode (50) configured to be placed proximal to the fluid output; and
an electric power source for supplying electrical power between the first and second electrodes;
wherein the electrical power source (22) is configured to provide intermittent electrical pulses between the first and second electrode such that electrolysis occurs at the surface of the metallic device, wherein the potential difference between the first and second electrode is at least 1V; **characterized in that** the current applied in each electrical pulse is up to 2A, and wherein each electrical pulse has a duration of less than 1 second.

6. The apparatus according to claim 5, wherein the electrical power source is configured to provide intermittent electrical pulses with a potential difference between 1V and 50V, and preferably between 10V and 30V, and a current between the first and second electrodes between 0.5A and 1A.

7. The apparatus according to claim 5 or 6, wherein the electrical power source is configured to provide each electrical pulse with a duration between 0.05 and 0.2 seconds.

8. The apparatus according to any of claims 5 to 7, further comprising a controller configured to control the supply of electrolytic fluid from the source of fluid to the fluid output.

9. The apparatus according to any of claims 5 to 8, wherein the fluid output and/or the first electrode and/or the second electrode are removably connectable to the apparatus via a detachment mechanism.

10. The apparatus according to any of claims 5 to 9, wherein the fluid output comprises a tubular body, optionally wherein the tubular body is curved so that the longitudinal axis of the tubular body at a proximal end is offset from the longitudinal axis of the tubular body at a distal end by between greater than 0 and 90 degrees.

11. The apparatus according to claim 10, wherein the fluid output comprises a porous sponge or a conical cap at its distal end.

12. The apparatus according to any of claims 5 to 11, wherein the first or second electrode and the fluid output are provided on a single detachable body connectable to the apparatus.

13. The apparatus according to claim 12, wherein both the first and second electrode are provided on the single detachable body.

14. The apparatus according to any one of claims 5 to 13 wherein the power source is configured to supply an intermittent direct current between the first and second electrodes.

15. The apparatus according to any one of claims 5 to 14, further comprising a source of electrolytic fluid connected to the fluid applicator, wherein the fluid is a saline solution; optionally wherein the fluid comprises one or more of a disinfecting agent, antibacterial agent or antibiotic agent.

## Patentansprüche

1. *In-vitro*-Verfahren zur Entfernung von Biofilm aus, oder zur Verringerung der bakteriellen Adhäsion zu, einer metallischen medizinischen Vorrichtung, welche extern zum menschlichen oder tierischen Körper ist, umfassend:
das Verbinden einer ersten Elektrode (20) mit einer Oberfläche der medizinischen Vorrichtung;
das Aussetzen einer Zielfläche auf der Oberfläche der medizinischen Vorrichtung zu einem Elektrolytfluid;
das Platzieren einer zweiten Elektrode (50) proximal zur Zielfläche;
das Bereitstellen von elektrischer Leistung zwischen der ersten und der zweiten Elektrode, sodass eine Elektrolyse im Elektrolytfluid stattfindet;
wobei die elektrische Leistung eine Vielzahl von elektrischen Impulsen umfasst, wobei der Potenzialunterschied zwischen der ersten und der zweiten Elektrode mindestens 1 V ist;
**dadurch gekennzeichnet, dass** der angelegte Strom in jedem elektrischen Impuls bis zu 2 A ist, und wobei jeder elektrische Impuls eine Dauer von weniger als 1 Sekunde hat.

2. Verfahren nach Anspruch 1, wobei der Schritt des Bereitstellens von elektrischer Leistung zwischen der ersten und der zweiten Elektrode das Bereitstellen eines Potenzialunterschieds zwischen 1 V und 50 V, vorzugsweise zwischen 10 V und 30 V, umfasst, und der Schritt des Bereitstellens von elektrischer Leistung zwischen der ersten und der zweiten Elektrode zusätzlich das Bereitstellen eines Stroms zwischen der ersten und der zweiten Elektrode zwischen 0,5 A und 1 A umfasst.

3. Verfahren nach Anspruch 1 oder 2, wobei jeder elektrische Impuls eine Dauer zwischen 0,05 und 0,2 Sekunden hat.

4. Verfahren nach einem der Ansprüche 1 bis 3, wobei die zweite Elektrode eine Anode ist, welche ein Opfermetall, vorzugsweise aus Silber bestehend, umfasst, und/oder wobei das Elektrolytfluid eins oder mehr aus einem Desinfektionsmittel, einem antibakteriellen Mittel oder einem antibiotischen Mittel umfasst; oder wobei die zweite Elektrode eine Anode ist, welche ein nicht korrodierbares Metall und frei von Opfermetallen umfasst, wobei das Verfahren zusätzlich das Isolieren von Bakterien aus dem Elektrolytfluid umfasst, nachdem die elektrische Leistung bereitgestellt wird.

5. Gerät zur Entfernung von Biofilm aus einer metallischen medizinischen Vorrichtung, umfassend:
einen Fluidausgang, welcher mit einer Quelle von Elektrolytfluid verbindbar ist, wobei der Fluidausgang zum Zuführen des Elektrolytfluids zum Waschen einer Zielfläche der medizinischen Vorrichtung ist;
eine erste Elektrode (20) zur elektrischen Verbindung mit der medizinischen Vorrichtung;
eine zweite Elektrode (50), welche dazu ausgebildet ist, proximal zum Fluidausgang platziert zu werden; und
eine elektrische Leistungsquelle zum Zuführen von elektrischer Leistung zwischen der ersten und der zweiten Elektrode;
wobei die elektrische Leistungsquelle (22) dazu ausgebildet ist, intermittierende elektrische Impulse zwischen der ersten und der zweiten Elektrode bereitzustellen, sodass eine Elektrolyse an der Oberfläche der metallischen Vorrichtung stattfindet, wobei der Potenzialunterschied zwischen der ersten und der zweiten Elektrode mindestens 1 V ist;
**dadurch gekennzeichnet, dass** der angelegte Strom in jedem elektrischen Impuls bis zu 2 A ist, und wobei jeder elektrische Impuls eine Dauer von weniger als 1 Sekunde hat.

6. Gerät nach Anspruch 5, wobei die elektrische Leistungsquelle dazu ausgebildet ist, intermittierende elektrische Impulse mit einem Potenzialunterschied zwischen 1 V und 50 V, und vorzugsweise zwischen 10 V und 30 V, und einen Strom zwischen der ersten und der zweiten Elektrode zwischen 0,5 A und 1 A bereitzustellen.

7. Gerät nach Anspruch 5 oder 6, wobei die elektrische Leistungsquelle dazu ausgebildet ist, jeden elektrischen Impuls mit einer Dauer zwischen 0,05 und 0,2 Sekunden bereitzustellen.

8. Gerät nach einem der Ansprüche 5 bis 7, zusätzlich umfassend eine Steuerung, welche dazu ausgebildet ist, das Zuführen von Elektrolytfluid von der Quelle des Fluids aus zum Fluidausgang zu steuern.

9. Gerät nach einem der Ansprüche 5 bis 8, wobei der Fluidausgang und/oder die erste Elektrode und/oder die zweite Elektrode mit dem Gerät mittels eines Entkopplungsmechanismus lösbar verbindbar sind.

10. Gerät nach einem der Ansprüche 5 bis 9, wobei der Fluidausgang einen rohrförmigen Körper umfasst, wobei wahlweise der rohrförmige Körper derart gekrümmt ist, dass die Längsachse des rohrförmigen Körpers an einem proximalen Ende in Bezug auf die Längsachse des rohrförmigen Körpers an einem distalen Ende mehr als zwischen 0 und 90 Grad versetzt ist.

11. Gerät nach Anspruch 10, wobei der Fluidausgang einen porösen Schwamm oder eine konische Kappe an dessen distalen Ende umfasst.

12. Gerät nach einem der Ansprüche 5 bis 11, wobei die erste oder die zweite Elektrode und der Fluidausgang auf einem einzigen entkoppelbaren Körper vorgesehen sind, welcher mit dem Gerät verbindbar ist.

13. Gerät nach Anspruch 12, wobei sowohl die erste als auch die zweite Elektrode auf dem einzigen entkoppelbaren Körper vorgesehen sind.

14. Gerät nach einem der Ansprüche 5 bis 13, wobei die Leistungsquelle dazu ausgebildet ist, einen intermittierenden direkten Strom zwischen der ersten und der zweiten Elektrode zuzuführen.

15. Gerät nach einem der Ansprüche 5 bis 14, zusätzlich umfassend eine Quelle von Elektrolytfluid, welche mit dem Fluidapplikator verbunden ist, wobei das Fluid eine Salzlösung ist; wobei wahlweise das Fluid eins oder mehrere aus einem Desinfektionsmittel, einem antibakteriellen Mittel oder einem antibiotischen Mittel umfasst.

## Revendications

1. Procédé *in vitro* d'élimination de biofilm de, ou réduction de l'adhésion bactérienne à, un dispositif médical métallique externe au corps humain ou animal, comprenant :
connecter une première électrode (20) à une surface du dispositif médical ;
exposer une zone cible sur la surface du dispositif médical à un fluide électrolytique ;
placer une deuxième électrode (50) de manière proximale à la zone cible ;
fournir de la puissance électrique entre la première et la deuxième électrode afin de produire une électrolyse dans le fluide électrolytique ;
dans lequel la puissance électrique comprend une pluralité d'impulsions électriques, dans lequel la différence de potentiel entre la première et la deuxième électrode est d'au moins 1 V ;
**caractérisé en ce que** le courant appliqué dans chaque impulsion électrique peut atteindre 2 A, et dans lequel chaque impulsion présente une durée de moins de 1 seconde.

2. Procédé selon la revendication 1, dans lequel l'étape de fournir de la puissance électrique entre la première et la deuxième électrode comprend fournir une différence de potentiel comprise entre 1 V et 50 V, de préférence entre 10 V et 30 V, et l'étape de fournir de la puissance électrique entre la première et la deuxième électrode comprend en outre fournir un courant entre la première et la deuxième électrode compris entre 0,5 A et 1 A.

3. Procédé selon la revendication 1 ou 2, dans lequel chaque impulsion électrique présente une durée comprise entre 0,05 et 0,2 seconds.

4. Procédé selon l'une quelconque des revendications 1 à 3, dans lequel la deuxième électrode est une anode comprenant un métal sacrificiel, de préférence composé de l'argent, et/ou dans lequel le fluide électrolytique comprend un ou plusieurs parmi un agent désinfectant, un agent antibactérien ou un agent antibiotique ; ou dans lequel la deuxième électrode est une anode comprenant un métal non corrodable et elle est libre de métaux sacrificiels, dans lequel le procédé comprend en outre l'isolement de bactéries à partir du fluide électrolytique après avoir fourni la puissance électrique.

5. Appareil d'élimination de biofilm d'un dispositif médical métallique, comprenant :
une sortie de fluide pouvant reliée à une source de fluide électrolytique, la sortie de fluide servant à alimenter du fluide électrolytique pour laver une zone cible du dispositif médical ;
une première électrode (20) pour être connectée électriquement au dispositif médical ;
une deuxième électrode (50) configurée pour être placée de manière proximale à la sortie de fluide ; et
une source de puissance électrique pour alimenter de la puissance électrique entre la première et la deuxième électrode ;
dans lequel la source de puissance électrique (22) est configurée pour fournir des impulsions électriques intermittentes entre la première et la deuxième électrode afin de produire une électrolyse dans la surface du dispositif métallique, dans lequel la différence de potentiel entre la première et la deuxième électrode est d'au moins 1 V ;
**caractérisé en ce que** le courant appliqué dans chaque impulsion électrique peut atteindre 2 A, et dans lequel chaque impulsion électrique présente une durée de moins de 1 seconde.

6. Appareil selon la revendication 5, dans lequel la source de puissance électrique est configurée pour fournir des impulsions électriques intermittentes avec une différence de potentiel comprise entre 1 V et 50 V, et de préférence entre 10 V et 30 V, et un courant entre la première et la deuxième électrode compris entre 0,5 A et 1 A.

7. Appareil selon la revendication 5 ou 6, dans lequel la source de puissance électrique est configurée pour fournir chaque impulsion électrique avec une durée comprise entre 0,05 et 0,2 secondes.

8. Appareil selon l'une quelconque des revendications 5 à 7, comprenant en outre un contrôleur configuré pour contrôler l'alimentation de fluide électrolytique à partir de la source de fluide jusqu'à la sortie de fluide.

9. Appareil selon l'une quelconque des revendications 5 à 8, dans lequel la sortie de fluide et/ou la première électrode et/ou la deuxième électrode peuvent être reliées de manière amovible à l'appareil par le biais d'un mécanisme de détachement.

10. Appareil selon l'une quelconque des revendications 5 à 9, dans lequel la sortie de fluide comprend un corps tubulaire, optionnellement dans lequel le corps tubulaire est courbé de sorte que l'axe longitudinal du corps tubulaire dans une extrémité proximale est décalé par rapport à l'axe longitudinal du corps tubulaire dans une extrémité distale d'entre plus de 0 et 90 degrés.

11. Appareil selon la revendication 10, dans lequel la sortie de fluide comprend une éponge poreuse ou un capuchon conique dans son extrémité distale.

12. Appareil selon l'une quelconque des revendications 5 à 11, dans lequel la première ou la deuxième électrode et la sortie de fluide sont fournies sur un seul corps détachable pouvant être relié à l'appareil.

13. Appareil selon la revendication 12, dans lequel la première et la deuxième électrode sont toutes deux fournies sur le seul corps détachable.

14. Appareil selon l'une quelconque des revendications 5 à 13, dans lequel la source de puissance est configurée pour alimenter un courant continu intermittent entre la première et la deuxième électrode.

15. Appareil selon l'une quelconque des revendications 5 à 14, comprenant en outre une source de fluide électrolytique reliée à l'applicateur de fluide, dans lequel le fluide est une solution saline ; optionnellement dans lequel le fluide comprend un ou plusieurs parmi un agent désinfectant, un agent antibactérien ou un agent antibiotique.
